# EUROPEAN PATENT APPLICATION

(11) **EP 3 023 437 A1**
(43) Date of publication of application: **25.05.2016**
(21) Application number: 14194151.8
(22) Date of filing: 20.11.2014
(51) Int. Cl.: C07K 16/28, C07K 16/46, A61P 35/00

(54) **Bispecific antibodies against CD3epsilon and BCMA**

(71) Applicant: EngMab AG, 8832 Wilen (CH)
(72) Inventor: The designation of the inventor has not yet been filed
(74) Representative: Schreiner, Siegfried

(57) **Abstract**

An antibody light chain, comprising as CDRs CDR1L of SEQ ID NO:6, CDR2L of SEQ ID NO:7, and CDR3L of SEQ ID NO:8, is useful as common light chain in a bispecific antibody specifically binding to the extracellular domain of human BCMA and human CD3.Such bispecific antibody can be used as a medicament in the treatment of plasma cell disorders like Multiple Myeloma.

SEQUENCE LISTING

<110> ENGMAB AG

<120> BISPECIFIC ANTIBODIES AGAINST CD3EPSILON AND BCMA

<130> TCBCLC-EP

<160> 37

<170> BiSSAP 1.3

<210> 1
<211> 125
<212> PRT
<213> Mus musculus

<220>
<223> CH2527-VL7 VH

<400> 1
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Thr Tyr
            20                  25                  30
Ala Met Asn Trp Val Arg Gln Ala Pro Gly Lys Gly Leu Glu Trp Val
        35                  40                  45
Ser Arg Ile Arg Ser Lys Tyr Asn Asn Tyr Ala Thr Tyr Tyr Ala Asp
    50                  55                  60
Ser Val Lys Gly Arg Phe Thr Ile Ser Arg Asp Asp Ser Lys Asn Thr
65                  70                  75                  80
Leu Tyr Leu Gln Met Asn Ser Leu Arg Ala Glu Asp Thr Ala Val Tyr
                85                  90                  95
Tyr Cys Val Arg His Gly Asn Phe Gly Asn Ser Tyr Val Ser Trp Phe
            100                 105                 110
Ala Tyr Trp Gly Gln Gly Thr Leu Val Thr Val Ser Ser
        115                 120                 125

<210> 2
<211> 109
<212> PRT
<213> Mus musculus

<220>
<223> CH2527-VL7 VL

<400> 2
Gln Ala Val Val Thr Gln Glu Pro Ser Leu Thr Val Ser Pro Gly Gly
1               5                   10                  15
Thr Val Thr Leu Thr Cys Gly Ser Ser Thr Gly Ala Val Thr Thr Ser
            20                  25                  30
Asn Tyr Ala Asn Trp Val Gln Glu Lys Pro Gly Gln Ala Phe Arg Gly
        35                  40                  45
Leu Ile Gly Gly Thr Asn Lys Arg Ala Pro Gly Thr Pro Ala Arg Phe
    50                  55                  60
Ser Gly Ser Leu Leu Gly Gly Lys Ala Ala Leu Thr Leu Ser Gly Ala
65                  70                  75                  80
Gln Pro Glu Asp Glu Ala Glu Tyr Tyr Cys Ala Leu Trp Tyr Ser Asn
                85                  90                  95
Leu Trp Val Phe Gly Gly Gly Thr Lys Leu Thr Val Leu
            100                 105

<210> 3

<211> 5
<212> PRT
<213> Mus musculus

<220>
<223> CH2527-VL7 CDR1H

<400> 3
Thr Tyr Ala Met Asn
1               5

<210> 4
<211> 19
<212> PRT
<213> Mus musculus

<220>
<223> CH2527-VL7 CDR2H

<400> 4
Arg Ile Arg Ser Lys Tyr Asn Asn Tyr Ala Thr Tyr Tyr Ala Asp Ser
1               5                   10                  15
Val Lys Gly


<210> 5
<211> 14
<212> PRT
<213> Mus musculus

<220>
<223> CH2527-VL7 CDR3H

<400> 5
His Gly Asn Phe Gly Asn Ser Tyr Val Ser Trp Phe Ala Tyr
1               5                   10

<210> 6
<211> 14
<212> PRT
<213> Mus musculus

<220>
<223> CH2527-VL7 CDRL1

<400> 6
Gly Ser Ser Thr Gly Ala Val Thr Thr Ser Asn Tyr Ala Asn
1               5                   10

<210> 7
<211> 7
<212> PRT
<213> Mus musculus

<220>
<223> CH2527-VL7 CDR2L

<400> 7
Gly Thr Asn Lys Arg Ala Pro
1               5

<210> 8
<211> 9

<212> PRT
<213> Mus musculus

<220>
<223> CH2527-VL7 CDR3L

<400> 8
Ala Leu Trp Tyr Ser Asn Leu Trp Val
1               5

<210> 9
<211> 39
<212> DNA
<213> Artificial Sequence

<220>
<223> primer

<400> 9
aagcttggat ccatgttgca gatggctggg cagtgctcc                              39


<210> 10
<211> 49
<212> DNA
<213> Artificial Sequence

<220>
<223> primer

<400> 10
gaattcgcgg ccgctcatcc tttcactgaa ttggtcacac ttgcattac                   49


<210> 11
<211> 42
<212> DNA
<213> Artificial Sequence

<220>
<223> primer

<400> 11
acgttagatc tccactcagt cctgcatctt gttccagtta ac                          42


<210> 12
<211> 33
<212> DNA
<213> Artificial Sequence

<220>
<223> primer

<400> 12
aacgttgcgg ccgctagttt cacaaacccc agg                                    33


<210> 13
<211> 45
<212> DNA
<213> Artificial Sequence

<220>
<223> primer

<400> 13
gaattcaagc ttgccaccat gttgcagatg gctgggcagt gctcc                    45


<210> 14
<211> 48
<212> DNA
<213> Artificial Sequence

<220>
<223> primer

<400> 14
gaattctcta gattacctag cagaaattga tttctctatc tccgtagc                 48


<210> 15
<211> 119
<212> PRT
<213> Homo sapiens

<220>
<223> pSCHLI333 VH

<400> 15
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Met Phe Ser Ser Phe
            20                  25                  30
Gly Met Ser Trp Val Arg Gln Ala Pro Gly Gln Gly Leu Glu Trp Met
        35                  40                  45
Gly Trp Ile Tyr Pro Val Gly Gln Gly Thr Trp Tyr Ala Gln Lys Phe
    50                  55                  60
Gln Gly Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Val Ser Tyr Pro Pro Ser His Phe Asp Tyr Trp Gly Gln Gly
            100                 105                 110
Thr Leu Val Thr Val Ser Ser
        115

<210> 16
<211> 119
<212> PRT
<213> Homo sapiens

<220>
<223> pSCHLI372 VH

<400> 16
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asn Ser
            20                  25                  30
Gly Met Ile Trp Val Arg Gln Ala Pro Gly Lys Gly Leu Glu Trp Val
        35                  40                  45
Gly His Ile Arg Ser Lys Thr Asp Gly Gly Thr Thr Asp Tyr Ala Ala
    50                  55                  60
Pro Val Lys Gly Arg Phe Thr Ile Ser Arg Asp Asp Ser Lys Asn Thr

65                  70                  75                  80
Leu Tyr Leu Gln Met Asn Ser Leu Lys Thr Glu Asp Thr Ala Val Tyr
                85                  90                  95
Tyr Cys Thr Thr Gly Gly Ser Gly Ser Phe Asp Tyr Trp Gly Gln Gly
            100                 105                 110
Thr Leu Val Thr Val Ser Ser
        115

<210> 17
<211> 120
<212> PRT
<213> Homo sapiens

<220>
<223> pSCHLI373 VH

<400> 17
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Ser Phe Ser Asn Ser
            20                  25                  30
Trp Met Asn Trp Val Arg Gln Ala Pro Gly Lys Gly Leu Glu Trp Val
        35                  40                  45
Gly Thr Ile Arg Gln Lys Thr Tyr Gly Gly Thr Thr Asp Tyr Ala Ala
    50                  55                  60
Pro Val Lys Gly Arg Phe Thr Ile Ser Arg Asp Asp Ser Lys Asn Thr
65                  70                  75                  80
Leu Tyr Leu Gln Met Asn Ser Leu Lys Thr Glu Asp Thr Ala Val Tyr
                85                  90                  95
Tyr Cys Thr Thr Gly Gly Leu Phe Gly Tyr Trp Asp Tyr Trp Gly Gln
            100                 105                 110
Gly Thr Leu Val Thr Val Ser Ser
        115                 120

<210> 18
<211> 10
<212> PRT
<213> Homo sapiens

<400> 18
Gly Tyr Met Phe Ser Ser Phe Gly Met Ser
1               5                   10

<210> 19
<211> 10
<212> PRT
<213> Homo sapiens

<400> 19
Gly Phe Thr Phe Ser Asn Ser Gly Met Ile
1               5                   10

<210> 20
<211> 10
<212> PRT
<213> Homo sapiens

<400> 20
Gly Phe Ser Phe Ser Asn Ser Trp Met Asn
1               5                   10

<210> 21
<211> 17

<212> PRT
<213> Homo sapiens

<400> 21
Trp Ile Tyr Pro Val Gly Gln Gly Thr Trp Tyr Ala Gln Lys Phe Gln
1               5                   10                  15
Gly


<210> 22
<211> 19
<212> PRT
<213> Homo sapiens

<400> 22
His Ile Arg Ser Lys Thr Asp Gly Gly Thr Thr Asp Tyr Ala Ala Pro
1               5                   10                  15
Val Lys Gly


<210> 23
<211> 19
<212> PRT
<213> Homo sapiens

<400> 23
Thr Ile Arg Gln Lys Thr Tyr Gly Gly Thr Thr Asp Tyr Ala Ala Pro
1               5                   10                  15
Val Lys Gly


<210> 24
<211> 10
<212> PRT
<213> Homo sapiens

<400> 24
Val Ser Tyr Pro Pro Ser His Phe Asp Tyr
1               5                   10

<210> 25
<211> 8
<212> PRT
<213> Homo sapiens

<400> 25
Gly Gly Ser Gly Ser Phe Asp Tyr
1               5

<210> 26
<211> 9
<212> PRT
<213> Homo sapiens

<400> 26
Gly Gly Leu Phe Gly Tyr Trp Asp Tyr
1               5

<210> 27
<211> 688
<212> PRT
<213> Artificial Sequence

<220>
<223> pSCHLI333 VH_CH1 x CD3 VH_CH1 Fc knob LALA PG

<400> 27
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Met Phe Ser Ser Phe
            20                  25                  30
Gly Met Ser Trp Val Arg Gln Ala Pro Gly Gln Gly Leu Glu Trp Met
        35                  40                  45
Gly Trp Ile Tyr Pro Val Gly Gln Gly Thr Trp Tyr Ala Gln Lys Phe
    50                  55                  60
Gln Gly Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Val Ser Tyr Pro Pro Ser His Phe Asp Tyr Trp Gly Gln Gly
            100                 105                 110
Thr Leu Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Gly
    210                 215                 220
Gly Gly Gly Ser Gly Gly Gly Gly Ser Glu Val Gln Leu Leu Glu Ser
225                 230                 235                 240
Gly Gly Gly Leu Val Gln Pro Gly Gly Ser Leu Arg Leu Ser Cys Ala
                245                 250                 255
Ala Ser Gly Phe Thr Phe Ser Thr Tyr Ala Met Asn Trp Val Arg Gln
            260                 265                 270
Ala Pro Gly Lys Gly Leu Glu Trp Val Ser Arg Ile Arg Ser Lys Tyr
        275                 280                 285
Asn Asn Tyr Ala Thr Tyr Tyr Ala Asp Ser Val Lys Gly Arg Phe Thr
    290                 295                 300
Ile Ser Arg Asp Asp Ser Lys Asn Thr Leu Tyr Leu Gln Met Asn Ser
305                 310                 315                 320
Leu Arg Ala Glu Asp Thr Ala Val Tyr Tyr Cys Val Arg His Gly Asn
                325                 330                 335
Phe Gly Asn Ser Tyr Val Ser Trp Phe Ala Tyr Trp Gly Gln Gly Thr
            340                 345                 350
Leu Val Thr Val Ser Ser Ala Ser Thr Lys Gly Pro Ser Val Phe Pro
        355                 360                 365
Leu Ala Pro Ser Ser Lys Ser Thr Ser Gly Gly Thr Ala Ala Leu Gly
    370                 375                 380
Cys Leu Val Lys Asp Tyr Phe Pro Glu Pro Val Thr Val Ser Trp Asn
385                 390                 395                 400
Ser Gly Ala Leu Thr Ser Gly Val His Thr Phe Pro Ala Val Leu Gln
                405                 410                 415
Ser Ser Gly Leu Tyr Ser Leu Ser Ser Val Val Thr Val Pro Ser Ser
            420                 425                 430
Ser Leu Gly Thr Gln Thr Tyr Ile Cys Asn Val Asn His Lys Pro Ser
        435                 440                 445
Asn Thr Lys Val Asp Lys Lys Val Glu Pro Lys Ser Cys Asp Lys Thr
    450                 455                 460
His Thr Cys Pro Pro Cys Pro Ala Pro Glu Ala Ala Gly Gly Pro Ser

465                 470                 475                 480
Val Phe Leu Phe Pro Pro Lys Pro Lys Asp Thr Leu Met Ile Ser Arg
                485                 490                 495
Thr Pro Glu Val Thr Cys Val Val Val Asp Val Ser His Glu Asp Pro
            500                 505                 510
Glu Val Lys Phe Asn Trp Tyr Val Asp Gly Val Glu Val His Asn Ala
        515                 520                 525
Lys Thr Lys Pro Arg Glu Glu Gln Tyr Asn Ser Thr Tyr Arg Val Val
    530                 535                 540
Ser Val Leu Thr Val Leu His Gln Asp Trp Leu Asn Gly Lys Glu Tyr
545                 550                 555                 560
Lys Cys Lys Val Ser Asn Lys Ala Leu Gly Ala Pro Ile Glu Lys Thr
                565                 570                 575
Ile Ser Lys Ala Lys Gly Gln Pro Arg Glu Pro Gln Val Tyr Thr Leu
            580                 585                 590
Pro Pro Cys Arg Asp Glu Leu Thr Lys Asn Gln Val Ser Leu Trp Cys
        595                 600                 605
Leu Val Lys Gly Phe Tyr Pro Ser Asp Ile Ala Val Glu Trp Glu Ser
    610                 615                 620
Asn Gly Gln Pro Glu Asn Asn Tyr Lys Thr Thr Pro Pro Val Leu Asp
625                 630                 635                 640
Ser Asp Gly Ser Phe Phe Leu Tyr Ser Lys Leu Thr Val Asp Lys Ser
                645                 650                 655
Arg Trp Gln Gln Gly Asn Val Phe Ser Cys Ser Val Met His Glu Ala
            660                 665                 670
Leu His Asn His Tyr Thr Gln Lys Ser Leu Ser Leu Ser Pro Gly Lys
        675                 680                 685

<210> 28
<211> 449
<212> PRT
<213> Artificial Sequence

<220>
<223> pSCHLI333 HC hole LALA PG

<400> 28
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Met Phe Ser Ser Phe
            20                  25                  30
Gly Met Ser Trp Val Arg Gln Ala Pro Gly Gln Gly Leu Glu Trp Met
        35                  40                  45
Gly Trp Ile Tyr Pro Val Gly Gln Gly Thr Trp Tyr Ala Gln Lys Phe
    50                  55                  60
Gln Gly Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Val Ser Tyr Pro Pro Ser His Phe Asp Tyr Trp Gly Gln Gly
            100                 105                 110
Thr Leu Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Ala Ala Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Gly Ala Pro Ile Glu Lys
                325                 330                 335
Thr Ile Ser Lys Ala Lys Gly Gln Pro Arg Glu Pro Gln Val Cys Thr
            340                 345                 350
Leu Pro Pro Ser Arg Asp Glu Leu Thr Lys Asn Gln Val Ser Leu Ser
        355                 360                 365
Cys Ala Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Val Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys


<210> 29
<211> 215
<212> PRT
<213> Artificial Sequence

<220>
<223> CD3 LC (CLC)

<400> 29
Gln Ala Val Val Thr Gln Glu Pro Ser Leu Thr Val Ser Pro Gly Gly
1               5                   10                  15
Thr Val Thr Leu Thr Cys Gly Ser Ser Thr Gly Ala Val Thr Thr Ser
            20                  25                  30
Asn Tyr Ala Asn Trp Val Gln Glu Lys Pro Gly Gln Ala Phe Arg Gly
        35                  40                  45
Leu Ile Gly Gly Thr Asn Lys Arg Ala Pro Gly Thr Pro Ala Arg Phe
    50                  55                  60
Ser Gly Ser Leu Leu Gly Gly Lys Ala Ala Leu Thr Leu Ser Gly Ala
65                  70                  75                  80
Gln Pro Glu Asp Glu Ala Glu Tyr Tyr Cys Ala Leu Trp Tyr Ser Asn
                85                  90                  95
Leu Trp Val Phe Gly Gly Gly Thr Lys Leu Thr Val Leu Gly Gln Pro
            100                 105                 110
Lys Ala Ala Pro Ser Val Thr Leu Phe Pro Pro Ser Ser Glu Glu Leu
        115                 120                 125
Gln Ala Asn Lys Ala Thr Leu Val Cys Leu Ile Ser Asp Phe Tyr Pro
    130                 135                 140
Gly Ala Val Thr Val Ala Trp Lys Ala Asp Ser Ser Pro Val Lys Ala

145                 150                 155                 160
Gly Val Glu Thr Thr Thr Pro Ser Lys Gln Ser Asn Asn Lys Tyr Ala
                165                 170                 175
Ala Ser Ser Tyr Leu Ser Leu Thr Pro Glu Gln Trp Lys Ser His Arg
            180                 185                 190
Ser Tyr Ser Cys Gln Val Thr His Glu Gly Ser Thr Val Glu Lys Thr
        195                 200                 205
Val Ala Pro Thr Glu Cys Ser
    210                 215

<210> 30
<211> 688
<212> PRT
<213> Artificial Sequence

<220>
<223> pSCHLI372 VH_CH1 x CD3 VH_CH1 Fc knob LALA PG

<400> 30
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asn Ser
            20                  25                  30
Gly Met Ile Trp Val Arg Gln Ala Pro Gly Lys Gly Leu Glu Trp Val
        35                  40                  45
Gly His Ile Arg Ser Lys Thr Asp Gly Gly Thr Thr Asp Tyr Ala Ala
    50                  55                  60
Pro Val Lys Gly Arg Phe Thr Ile Ser Arg Asp Asp Ser Lys Asn Thr
65                  70                  75                  80
Leu Tyr Leu Gln Met Asn Ser Leu Lys Thr Glu Asp Thr Ala Val Tyr
                85                  90                  95
Tyr Cys Thr Thr Gly Gly Ser Gly Ser Phe Asp Tyr Trp Gly Gln Gly
            100                 105                 110
Thr Leu Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Gly
    210                 215                 220
Gly Gly Gly Ser Gly Gly Gly Gly Ser Glu Val Gln Leu Leu Glu Ser
225                 230                 235                 240
Gly Gly Gly Leu Val Gln Pro Gly Gly Ser Leu Arg Leu Ser Cys Ala
                245                 250                 255
Ala Ser Gly Phe Thr Phe Ser Thr Tyr Ala Met Asn Trp Val Arg Gln
            260                 265                 270
Ala Pro Gly Lys Gly Leu Glu Trp Val Ser Arg Ile Arg Ser Lys Tyr
        275                 280                 285
Asn Asn Tyr Ala Thr Tyr Tyr Ala Asp Ser Val Lys Gly Arg Phe Thr
    290                 295                 300
Ile Ser Arg Asp Asp Ser Lys Asn Thr Leu Tyr Leu Gln Met Asn Ser
305                 310                 315                 320
Leu Arg Ala Glu Asp Thr Ala Val Tyr Tyr Cys Val Arg His Gly Asn
                325                 330                 335
Phe Gly Asn Ser Tyr Val Ser Trp Phe Ala Tyr Trp Gly Gln Gly Thr
            340                 345                 350

Leu Val Thr Val Ser Ser Ala Ser Thr Lys Gly Pro Ser Val Phe Pro
        355                 360                 365
Leu Ala Pro Ser Ser Lys Ser Thr Ser Gly Gly Thr Ala Ala Leu Gly
    370                 375                 380
Cys Leu Val Lys Asp Tyr Phe Pro Glu Pro Val Thr Val Ser Trp Asn
385                 390                 395                 400
Ser Gly Ala Leu Thr Ser Gly Val His Thr Phe Pro Ala Val Leu Gln
                405                 410                 415
Ser Ser Gly Leu Tyr Ser Leu Ser Ser Val Val Thr Val Pro Ser Ser
            420                 425                 430
Ser Leu Gly Thr Gln Thr Tyr Ile Cys Asn Val Asn His Lys Pro Ser
        435                 440                 445
Asn Thr Lys Val Asp Lys Lys Val Glu Pro Lys Ser Cys Asp Lys Thr
    450                 455                 460
His Thr Cys Pro Pro Cys Pro Ala Pro Glu Ala Ala Gly Gly Pro Ser
465                 470                 475                 480
Val Phe Leu Phe Pro Pro Lys Pro Lys Asp Thr Leu Met Ile Ser Arg
                485                 490                 495
Thr Pro Glu Val Thr Cys Val Val Val Asp Val Ser His Glu Asp Pro
            500                 505                 510
Glu Val Lys Phe Asn Trp Tyr Val Asp Gly Val Glu Val His Asn Ala
        515                 520                 525
Lys Thr Lys Pro Arg Glu Glu Gln Tyr Asn Ser Thr Tyr Arg Val Val
    530                 535                 540
Ser Val Leu Thr Val Leu His Gln Asp Trp Leu Asn Gly Lys Glu Tyr
545                 550                 555                 560
Lys Cys Lys Val Ser Asn Lys Ala Leu Gly Ala Pro Ile Glu Lys Thr
                565                 570                 575
Ile Ser Lys Ala Lys Gly Gln Pro Arg Glu Pro Gln Val Tyr Thr Leu
            580                 585                 590
Pro Pro Cys Arg Asp Glu Leu Thr Lys Asn Gln Val Ser Leu Trp Cys
        595                 600                 605
Leu Val Lys Gly Phe Tyr Pro Ser Asp Ile Ala Val Glu Trp Glu Ser
    610                 615                 620
Asn Gly Gln Pro Glu Asn Asn Tyr Lys Thr Thr Pro Pro Val Leu Asp
625                 630                 635                 640
Ser Asp Gly Ser Phe Phe Leu Tyr Ser Lys Leu Thr Val Asp Lys Ser
                645                 650                 655
Arg Trp Gln Gln Gly Asn Val Phe Ser Cys Ser Val Met His Glu Ala
            660                 665                 670
Leu His Asn His Tyr Thr Gln Lys Ser Leu Ser Leu Ser Pro Gly Lys
        675                 680                 685

<210> 31
<211> 449
<212> PRT
<213> Artificial Sequence

<220>
<223> pSCHLI372 HC hole LALA PG

<400> 31
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asn Ser
            20                  25                  30
Gly Met Ile Trp Val Arg Gln Ala Pro Gly Lys Gly Leu Glu Trp Val
        35                  40                  45
Gly His Ile Arg Ser Lys Thr Asp Gly Gly Thr Thr Asp Tyr Ala Ala
    50                  55                  60
Pro Val Lys Gly Arg Phe Thr Ile Ser Arg Asp Asp Ser Lys Asn Thr

65                  70                  75                  80
Leu Tyr Leu Gln Met Asn Ser Leu Lys Thr Glu Asp Thr Ala Val Tyr
                85                  90                  95
Tyr Cys Thr Thr Gly Gly Ser Gly Ser Phe Asp Tyr Trp Gly Gln Gly
            100                 105                 110
Thr Leu Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Ala Ala Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Gly Ala Pro Ile Glu Lys
                325                 330                 335
Thr Ile Ser Lys Ala Lys Gly Gln Pro Arg Glu Pro Gln Val Cys Thr
            340                 345                 350
Leu Pro Pro Ser Arg Asp Glu Leu Thr Lys Asn Gln Val Ser Leu Ser
        355                 360                 365
Cys Ala Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Val Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys

<210> 32
<211> 689
<212> PRT
<213> Artificial Sequence

<220>
<223> pSCHLI373 VH_CH1 x CD3 VH_CH1 Fc knob LALA PG

<400> 32
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Ser Phe Ser Asn Ser
            20                  25                  30

Trp Met Asn Trp Val Arg Gln Ala Pro Gly Lys Gly Leu Glu Trp Val
        35                  40                  45
Gly Thr Ile Arg Gln Lys Thr Tyr Gly Gly Thr Thr Asp Tyr Ala Ala
    50                  55                  60
Pro Val Lys Gly Arg Phe Thr Ile Ser Arg Asp Asp Ser Lys Asn Thr
65                  70                  75                  80
Leu Tyr Leu Gln Met Asn Ser Leu Lys Thr Glu Asp Thr Ala Val Tyr
                85                  90                  95
Tyr Cys Thr Thr Gly Gly Leu Phe Gly Tyr Trp Asp Tyr Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Gly Gly Gly Gly Ser Gly Gly Gly Gly Ser Glu Val Gln Leu Leu Glu
225                 230                 235                 240
Ser Gly Gly Gly Leu Val Gln Pro Gly Gly Ser Leu Arg Leu Ser Cys
                245                 250                 255
Ala Ala Ser Gly Phe Thr Phe Ser Thr Tyr Ala Met Asn Trp Val Arg
            260                 265                 270
Gln Ala Pro Gly Lys Gly Leu Glu Trp Val Ser Arg Ile Arg Ser Lys
        275                 280                 285
Tyr Asn Asn Tyr Ala Thr Tyr Tyr Ala Asp Ser Val Lys Gly Arg Phe
    290                 295                 300
Thr Ile Ser Arg Asp Asp Ser Lys Asn Thr Leu Tyr Leu Gln Met Asn
305                 310                 315                 320
Ser Leu Arg Ala Glu Asp Thr Ala Val Tyr Tyr Cys Val Arg His Gly
                325                 330                 335
Asn Phe Gly Asn Ser Tyr Val Ser Trp Phe Ala Tyr Trp Gly Gln Gly
            340                 345                 350
Thr Leu Val Thr Val Ser Ser Ala Ser Thr Lys Gly Pro Ser Val Phe
        355                 360                 365
Pro Leu Ala Pro Ser Ser Lys Ser Thr Ser Gly Gly Thr Ala Ala Leu
    370                 375                 380
Gly Cys Leu Val Lys Asp Tyr Phe Pro Glu Pro Val Thr Val Ser Trp
385                 390                 395                 400
Asn Ser Gly Ala Leu Thr Ser Gly Val His Thr Phe Pro Ala Val Leu
                405                 410                 415
Gln Ser Ser Gly Leu Tyr Ser Leu Ser Ser Val Val Thr Val Pro Ser
            420                 425                 430
Ser Ser Leu Gly Thr Gln Thr Tyr Ile Cys Asn Val Asn His Lys Pro
        435                 440                 445
Ser Asn Thr Lys Val Asp Lys Lys Val Glu Pro Lys Ser Cys Asp Lys
    450                 455                 460
Thr His Thr Cys Pro Pro Cys Pro Ala Pro Glu Ala Ala Gly Gly Pro
465                 470                 475                 480
Ser Val Phe Leu Phe Pro Pro Lys Pro Lys Asp Thr Leu Met Ile Ser
                485                 490                 495
Arg Thr Pro Glu Val Thr Cys Val Val Val Asp Val Ser His Glu Asp
            500                 505                 510
Pro Glu Val Lys Phe Asn Trp Tyr Val Asp Gly Val Glu Val His Asn
        515                 520                 525
Ala Lys Thr Lys Pro Arg Glu Glu Gln Tyr Asn Ser Thr Tyr Arg Val

530                 535                 540
Val Ser Val Leu Thr Val Leu His Gln Asp Trp Leu Asn Gly Lys Glu
545                 550                 555                 560
Tyr Lys Cys Lys Val Ser Asn Lys Ala Leu Gly Ala Pro Ile Glu Lys
                565                 570                 575
Thr Ile Ser Lys Ala Lys Gly Gln Pro Arg Glu Pro Gln Val Tyr Thr
            580                 585                 590
Leu Pro Pro Cys Arg Asp Glu Leu Thr Lys Asn Gln Val Ser Leu Trp
        595                 600                 605
Cys Leu Val Lys Gly Phe Tyr Pro Ser Asp Ile Ala Val Glu Trp Glu
    610                 615                 620
Ser Asn Gly Gln Pro Glu Asn Asn Tyr Lys Thr Thr Pro Pro Val Leu
625                 630                 635                 640
Asp Ser Asp Gly Ser Phe Phe Leu Tyr Ser Lys Leu Thr Val Asp Lys
                645                 650                 655
Ser Arg Trp Gln Gln Gly Asn Val Phe Ser Cys Ser Val Met His Glu
            660                 665                 670
Ala Leu His Asn His Tyr Thr Gln Lys Ser Leu Ser Leu Ser Pro Gly
        675                 680                 685
Lys

<210> 33
<211> 450
<212> PRT
<213> Artificial Sequence

<220>
<223> pSCHLI373 HC hole LALA PG

<400> 33
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Ser Phe Ser Asn Ser
            20                  25                  30
Trp Met Asn Trp Val Arg Gln Ala Pro Gly Lys Gly Leu Glu Trp Val
        35                  40                  45
Gly Thr Ile Arg Gln Lys Thr Tyr Gly Gly Thr Thr Asp Tyr Ala Ala
    50                  55                  60
Pro Val Lys Gly Arg Phe Thr Ile Ser Arg Asp Asp Ser Lys Asn Thr
65                  70                  75                  80
Leu Tyr Leu Gln Met Asn Ser Leu Lys Thr Glu Asp Thr Ala Val Tyr
                85                  90                  95
Tyr Cys Thr Thr Gly Gly Leu Phe Gly Tyr Trp Asp Tyr Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Ala Ala Gly Gly
225                 230                 235                 240
Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Gly Ala Pro Ile Glu
                325                 330                 335
Lys Thr Ile Ser Lys Ala Lys Gly Gln Pro Arg Glu Pro Gln Val Cys
            340                 345                 350
Thr Leu Pro Pro Ser Arg Asp Glu Leu Thr Lys Asn Gln Val Ser Leu
        355                 360                 365
Ser Cys Ala Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Val Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450

<210> 34
<211> 116
<212> PRT
<213> Homo sapiens

<220>
<223> 83A10 VH

<400> 34
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
        35                  40                  45
Ser Ala Ile Ser Gly Ser Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Val Leu Gly Trp Phe Asp Tyr Trp Gly Gln Gly Thr Leu Val
            100                 105                 110
Thr Val Ser Ser
        115

<210> 35
<211> 5
<212> PRT
<213> Homo sapiens

<400> 35
Ser Tyr Ala Met Ser
1               5

<210> 36

<211> 17
<212> PRT
<213> Homo sapiens

<400> 36
Ala Ile Ser Gly Ser Gly Gly Ser Thr Tyr Tyr Ala Asp Ser Val Lys
1               5                   10                  15
Gly


<210> 37
<211> 7
<212> PRT
<213> Homo sapiens

<400> 37
Val Leu Gly Trp Phe Asp Tyr
1               5

## Description

The present invention relates to novel bispecific antibodies against CD3ε and BCMA, their manufacture and use.

### Background of the Invention

Human B cell maturation target, also known as BCMA; TR17_HUMAN, TNFRSF17 (UniProt Q02223), is a member of the tumor necrosis receptor superfamily that is preferentially expressed in differentiated plasma cells [Laabi et al. 1992; Madry et al. 1998]. BCMA is a non glycosylated type III transmembrane protein, which is involved in B cell maturation, growth and survival. BCMA is a receptor for two ligands of the TNF superfamily: APRIL (a proliferation-inducing ligand), the high-affinity ligand to BCMA and the B cell activation factor BAFF, the low-affinity ligand to BCMA (THANK, BlyS, B lymphocyte stimulator, TALL-1 and zTNF4). APRIL and BAFF show structural similarity and overlapping yet distinct receptor binding specificity. The negative regulator TACI also binds to both BAFF and APRIL. The coordinate binding of APRIL and BAFF to BCMA and/or TACI activates transcription factor NF-κB and increases the expression of pro-survival Bcl-2 family members (e.g. Bcl-2, Bcl-xL, Bcl-w, Mcl-1, A1) and the downregulation of pro-apoptotic factors (e.g. Bid, Bad, Bik, Bim, etc.), thus inhibiting apoptosis and promoting survival. This combined action promotes B cell differentiation, proliferation, survival and antibody production (as reviewed in Rickert RC et al., Immunol Rev (2011) 244 (1): 115-133).

The TCR/CD3 complex of T-lymphocytes consists of either a TCR alpha (α)/beta (β) or TCR gamma (γ)/delta (δ) heterodimer coexpressed at the cell surface with the invariant subunits of CD3 labeled gamma (γ), delta (δ), epsilon (ε), zeta (ζ), and eta (η). Human CD3ε is described under UniProt P07766 (CD3E_HUMAN). An anti CD3ε antibody described in the state of the art is SP34 (Yang SJ, The Journal of Immunology (1986) 137; 1097-1100). SP34 reacts with both primate and human CD3. SP34 is available from PharMingen. A further anti CD3 antibody described in the state of the art is UCHT-1 (see WO2000041474). A further anti CD3 antibody described in the state of the art is BC-3 (Fred Hutchinson Cancer Research Institute; used in Phase I/II trials of GvHD, Anasetti et al., Transplantation 54: 844 (1992)).

A wide variety of recombinant bispecific antibody formats have been developed in the recent past, e.g. by fusion of, e.g. an IgG antibody format and single chain domains (see Kontermann RE, mAbs 4:2, (2012) 1-16). Bispecific antibodies wherein the variable domains VL and VH or the constant domains CL and CH1 are replaced by each other are described in WO2009080251 and WO2009080252.

An approach to circumvent the problem of mispaired byproducts, which is known as 'knobs-into-holes', aims at forcing the pairing of two different antibody heavy chains by introducing mutations into the CH3 domains to modify the contact interface. On one chain bulky amino acids were replaced by amino acids with short side chains to create a 'hole'. Conversely, amino acids with large side chains were introduced into the other CH3 domain, to create a 'knob'. By coexpressing these two heavy chains (and two of said common light chains for use according to the invention, which have to be appropriate for both heavy chains), high yields of heterodimer formation ('knob-hole') versus homodimer formation ('hole-hole' or 'knob-knob') was observed (Ridgway JB, Presta LG, Carter P; and WO1996027011). The percentage of heterodimer could be further increased by remodeling the interaction surfaces of the two CH3 domains using a phage display approach and the introduction of a disulfide bridge to stabilize the heterodimers (Merchant A.M, et al, Nature Biotech 16 (1998) 677-681; Aτwell S, Ridgway JB, Wells JA, Carter P., J MoI Biol 270 (1997) 26-35). New approaches for the knobs-into-holes technology are described in e.g. in EP 1870459A1. Although this format appears very attractive, no data describing progression towards the clinic are currently available. One important constraint of this strategy is that the light chains of the two parent antibodies have to be identical to prevent mispairing and formation of inactive molecules. Thus this technique is not appropriate for easily developing recombinant, bispecific antibodies against two targets starting from two antibodies against the first and the second target, as either the heavy chains of these antibodies and/or said common light chains for use according to the invention have to be optimized. Xie, Z., et al, J Immunol Methods 286 (2005) 95-101 refers to a format of bispecific antibody using scFvs in combination with knobs-into-holes technology for the FC part. WO2012116927 and WO2010145792 mention exchanging the CH1 and CL domains. WO2009080254 mentions knob in hole constructs for producing bispecific antibodies. WO 2006093794 relates to heterodimeric protein binding compositions. WO199937791 describes multipurpose antibody derivatives. Morrison, S.L., et al., J. Immunol. 160 (1998) 2802-2808 refers to the influence of variable region domain exchange on the functional properties of IgG.

WO 201302362 relate to heterodimerized polypeptides. WO201312733 relates to polypeptides comprising heterodimeric Fc regions. WO2012131555 relates to engineered heterodimeric immunoglobulins. EP 2647707 relates to engineered hetero-dimeric immunoglobulins. WO2009080251, WO 2009080252, WO 2009080253, WO 2009080254 and Schaefer, W. et al, PNAS, 108 (2011) 11187-1191 relate to bivalent, bispecific IgG antibodies with a domain crossover.

Antibodies against BCMA are described e.g. in Gras M-P. et al. Int Immunol. 7 (1995) 1093-1106, WO200124811, WO200124812, WO2010104949 and WO2012163805. Antibodies against BCMA and their use for the treatment of lymphomas and multiple myeloma are mentioned e.g. in WO2002066516 and WO2010104949. WO2013154760 relates to chimeric antigen receptors (CAR) comprising a BCMA recognition moiety and a T-cell activation moiety.

Ryan, MC et al., Mol. Cancer Ther. 6 (2007) 3009-3018 relate to targeting of BCMA for plasma cell malignancies. Antibody SG1, with ligand blocking activity could promote cytotoxicity of multiple myeloma (MM) cell lines as naked antibodies or as antibody-drug conjugates (ADC). SG1, an inhibitory BCMA antibody, blocks APRIL-dependent activation of nuclear factor-κB in a dose-dependent manner in vitro. Cytotoxicity of SG1 was assessed as a naked antibody after chimerization with and without Fc mutations that enhance FcγRIIIA binding. Ryan also mentions antibody SG2 which does not significantly inhibit APRIL binding to BCMA. However SG2 showed a 20 fold higher IC₅₀ value as SG1 measured as cytotoxic activity of a drug conjugate against BCMA positive myeloma cell lines. Ryan conclude that BCMA antibodies can act on myeloma cell lines through multiple mechanisms that include inhibition of APRIL-dependent NF-κB activation, promotion of tumor cell lysis by natural killer cell-mediated ADCC activity, and induction of cytotoxicity by ADCs.

Bispecific antibodies against CD3 and BCMA are mentioned in WO2007117600, WO2009132058, WO2012066058, WO2012143498, and WO2013072415. WO2014122143 and WO2014122144 describe antibodies against BCMA and bispecific antibodies against CD3 and BCMA comprising certain CDRs and variable domains disclosed also in the present invention. A variable light chain VL of SEQ ID NO:2 and a VL comprising as CDRs CDR1L of SEQ ID NO:6, CDR2L of SEQ ID NO:7 are mentioned herein as light chains of an anti CD3 antibody.

Klein Ch., et al., mAbs 4:6, 653-663; 2012 mentions knobs into-holes (KiH) technology combined with a common light chain. Merchant AM. Et al., Nature Biotech 16, 677-681 (1998) relates to bispecific antibodies with identical light chains and remodeled heavy chains. Weidle UH et al., relates to avoiding of L-chain mispairing by the use of the "common light chain" principle that combines two binders that share one light chain but still have separate specificities. WO2007147901, WO2013184761, and WO2014124326 relate also to the common light chain technology.

Accordingly there is a need for bispecific antibodies against CD3ε and BCMA which can be produced in high yield no light chain mispairing, easily purified and show high affinity to BCMA and killing of BCMA-positive cells.

### Summary of the Invention

The inventors have recognized that the light chain of an anti-CD3 antibody (CH2527) which is used in a bispecific antibody specifically binding to human BCMA and human CD3 as described in WO2014122143 and WO2014122144 can be used as common light chain in such bispecific antibodies.

The invention relates to an antibody light chain, comprising as CDRs CDR1L of SEQ ID NO:6, CDR2L of SEQ ID NO:7, and CDR3L of SEQ ID NO:8, for use as common light chain in a bispecific antibody specifically binding to the extracellular domain of human BCMA (further named also as "BCMA") and human CD3 (further named also as "CD3"), wherein the heavy chain of the CD3 binding part comprises as CDRs CDR1H of SEQ ID NO:3, CDR2H of SEQ ID NO:4, and CDR3H of SEQ ID NO:5.

Preferably the invention relates to an antibody light chain, comprising as variable light chain domain VL a VL of SEQ ID NO:2, for use as common light chain in a bispecific antibody specifically binding to the extracellular domain of human BCMA and human CD3, wherein the heavy chain of the CD3 binding part comprises as CDRs CDR1H of SEQ ID NO:3, CDR2H of SEQ ID NO:4, and CDR3H of SEQ ID NO:5.

Preferably the invention relates to an antibody light chain of SEQ ID NO:29 for use as common light chain in a bispecific antibody specifically binding to the extracellular domain of human BCMA and human CD3, wherein the heavy chain of the CD3 binding part comprises as CDRs CDR1H of SEQ ID NO:3, CDR2H of SEQ ID NO:4, and CDR3H of SEQ ID NO:5.

The invention relates to the use of an antibody light chain, comprising as CDRs CDR1L of SEQ ID NO:6, CDR2L of SEQ ID NO:7, and CDR3L of SEQ ID NO:8, as common light chain in a bispecific antibody specifically binding to the extracellular domain of human BCMA (further named also as "BCMA") and human CD3 (further named also as "CD3"), wherein the heavy chain of the CD3 binding part comprises as CDRs CDR1H of SEQ ID NO:3, CDR2H of SEQ ID NO:4, and CDR3H of SEQ ID NO:5. Preferably the antibody light chain used is characterized in comprising as variable light chain domain VL a VL of SEQ ID NO:2. Preferably the antibody light chain used is characterized in comprising as light chain the light chain of SEQ ID NO:29

The invention relates to a bispecific antibody specifically binding to the extracellular domain of human BCMA and human CD3, wherein the heavy chain of the CD3 binding part comprises as CDRs CDR1H of SEQ ID NO:3, CDR2H of SEQ ID NO:4, and CDR3H of SEQ ID NO:5 and an antibody light chain, comprising as CDRs CDR1L of SEQ ID NO:6, CDR2L of SEQ ID NO:7, and CDR3L of SEQ ID NO:8 is the common light chain.

Preferably the invention relates to a bispecific antibody specifically binding to the extracellular domain of human BCMA and human CD3, wherein the heavy chain of the CD3 binding part comprises as CDRs CDR1H of SEQ ID NO:3, CDR2H of SEQ ID NO:4, and CDR3H of SEQ ID NO:5 and an antibody light chain comprising as variable light chain domain VL a VL of SEQ ID NO:2 is the common light chain.

Preferably the invention relates to a bispecific antibody specifically binding to the extracellular domain of human BCMA and human CD3, characterized in that the heavy chain of the CD3 binding part comprises as CDRs CDR1H of SEQ ID NO:3, CDR2H of SEQ ID NO:4, and CDR3H of SEQ ID NO:5 and an antibody light chain of SEQ ID NO:29 is the common light chain.

Preferably the heavy chain of the CD3 binding part comprises as variable heavy chain domain VH a VH of SEQ ID NO:1.

The constant light chain CL of the common light chain according to the invention is of kappa or lambda type, preferably of kappa type.

Preferably the heavy chain of the BCMA binding part comprises as CDRs a CDR set selected from the group consisting of:
a) CDR1H of SEQ ID NO:18, CDR2H of SEQ ID NO:21, and CDR3H of SEQ ID NO:24 (set pSCHLI333)
b) CDR1H of SEQ ID NO:19, CDR2H of SEQ ID NO:22, and CDR3H of SEQ ID NO:25 (set pSCHLI372)
c) CDR1H of SEQ ID NO:20, CDR2H of SEQ ID NO:23, and CDR3H of SEQ ID NO:26 (set pSCHLI373)
d) CDR1H of SEQ ID NO:35, CDR2H of SEQ ID NO:36, and CDR3H of SEQ ID NO:37 (set 83A10)

Preferably the heavy chain of the BCMA binding part comprises as variable heavy chain domain VH a VH of SEQ ID NO:15, 16, 17 or 34 (83A10)

Preferably the affinity to human BCMA of said bispecific antibody is 200 nM or lower, measured at an antibody concentration of 25 nM in presence of human BCMA Fc fusion at a concentration 500 nM or lower in an affinity setup surface plasmon resonance assay.

Preferably the potency (EC50) to kill BCMA-positive H929 cells (ATCC CRL-9068) of said bispecific antibody is measured as 2 nM or lower, when used at a concentrations of 100 nM and lower, in presence of human PBCMs and H929 cells at a E:T ratio of 10:1 for 24h, in redirected T-cell killing LDH release assay.

Preferably the affinity to human BCMA of said BCMA binding part, measured in an antibody of human IgG1 type, wherein the light chain is of SEQ ID NO:29, is 200 nM or lower at an antibody concentration of 25 nM in presence of human BCMA Fc fusion at a concentration of 500 nM or lower in an affinity setup surface plasmon resonance assay.

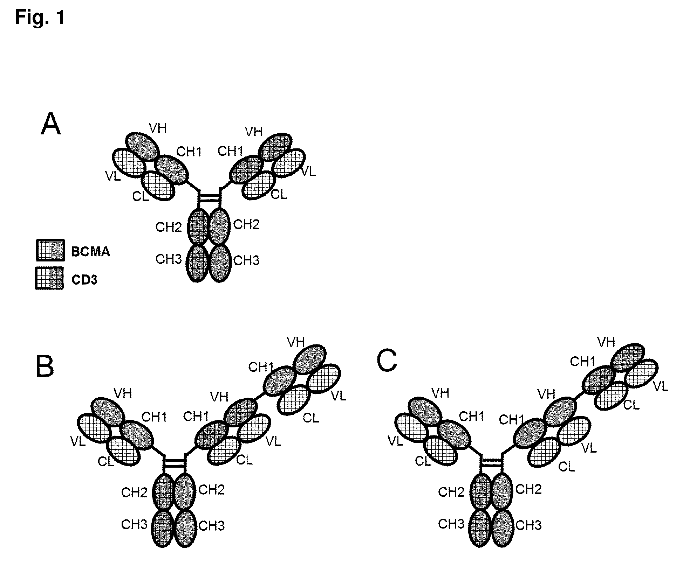

Preferably said bispecific antibody according to the invention consists of one Fab fragment of an antibody specifically binding to CD3 (further named also as "CD3-Fab"), and one Fab fragment of an antibody specifically binding to BCMA (further named also as "BCMA-Fab(s)") and a Fc part, wherein said CD3-Fab and said BCMA-Fab are linked via their C-termini to the hinge region of said Fc part and which said CD3-Fab and said BCMA-Fab comprise said common light chains for use according to the invention (Figure 1A).

Preferably said bispecific antibody according to the invention consists of two BCMA-Fabs, one CD3 Fab and a Fc part, wherein the first BCMA-Fab and the CD3-Fab are linked via their C-termini to the hinge region of said Fc part and said second BCMA-Fab is linked with its C-terminus to the N-terminus of said first BCMA-Fab. The CD3-Fab and BCMA-Fabs comprise said common light chains for use according to the invention (Figure 1B).

Preferably said bispecific antibody according to the invention consists of two BCMA-Fabs and a Fc part, wherein said BCMA-Fabs are linked via their C-termini to the hinge region of said Fc part and said CD3-Fab is linked with its C-terminus to the N-terminus of one BCMA-Fab. Said CD3-Fab and BCMA-Fabs comprise said common light chains for use according to the invention (Figure 1C).

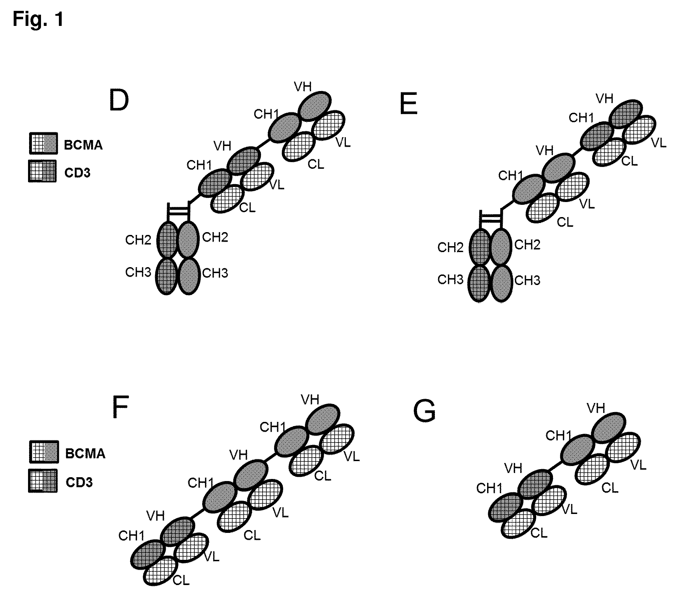

Preferably said bispecific antibody according to the invention consists of one CD3-Fab and one BCMA Fab, which is linked via its C-terminus to the hinge region of said Fc part and a BCMA-Fab, which is linked with its C-terminus to the N-terminus of said CD3-Fab. Said CD3-Fab and BCMA-Fab comprise said common light chains for use according to the invention (Figure 1D).

Preferably said bispecific antibody according to the invention consists of one BCMA-Fab and one CD3 Fab, wherein said BCMA Fab is linked via its C-terminus to the hinge region of said Fc part and said CD3-Fabis linked with its C-terminus to the N-terminus of said BCMA-Fab. Said CD3-Fab and BCMA-Fab comprise said common light chains for use according to the invention (Figure IE).

Preferably said bispecific antibody according to the invention consists of two BCMA-Fabs, wherein the first BCMA-Fab is linked via its C-terminus to the N-terminus of the second BCMA-Fab, and wherein the second BCMA-Fab is linked via its C-terminus to the N-terminus of the CD3-Fab. The CD3-Fab and both BCMA-Fabs comprise said common light chains for use according to the invention (Figure IF).

Preferably said bispecific antibody according to the invention consists of one BCMA-Fab and one CD3 Fab, wherein the BCMA Fab which is linked with its C-terminus to the N-terminus of the CD3-Fab. The CD3-Fab and BCMA-Fab comprise said common light chains for use according to the invention (Figure 1G).

In a preferred embodiment of the invention the antibody according to the invention consists of one CD3-Fab, and one BCMA-Fab and a Fc part, wherein the CD3-Fab and the BCMA-Fab are linked via their C-termini to the hinge region of said Fc part and a second BCMA-Fab, which is linked with its C-terminus to the N-terminus of the CD3-Fab. The CD3-Fab and the BCMA-Fab comprise said common light chains for use according to the invention (Figures 1B and 1C). Especially preferred is a bispecific antibody comprising BCMA-Fab-Fc-CD3-Fab-BCMA-Fab, wherein both BCMA-Fabs and the CD3-Fab comprise said common light chains for use according to the invention (Figures 1B). Especially preferred is that both BCMA-Fabs comprise as heavy chain CDRs the CDRHs of antibody pSCHLI333, or as VH the VH of pSCHLI333. Further preferred is that both BCMA-Fabs comprise as heavy chain CDRs the CDRHs of antibody pSCHLI372, or as VH the VH of pSCHLI372. Further preferred is that both BCMA-Fabs comprise as heavy chain CDRs the CDRHs of antibody pSCHLI373, or as VH the VH of pSCHLI373. Further preferred is that both BCMA-Fabs comprise as heavy chain CDRs the CDRs of antibody 83A10, as VH the VH of 83A10.

The Fab fragments are chemically linked together by the use of an appropriate linker according to the state of the art. Preferably a (Gly4-Ser1)3 linker is used (Desplancq DK et al., Protein Eng. 1994 Aug;7(8):1027-33 and Mack M. et al., PNAS July 18, 1995 vol. 92 no. 15 7021-7025). Linkage between two Fab fragments is performed between the heavy chains. Therefore the C-terminus of CH1 of a first Fab fragment is linked to the N-terminus of VH of the second Fab fragment. Linkage between a Fab fragment and the Fc part is performed according to the invention as linkage between CH1 and CH2.

The first and a second Fab fragment of an antibody specifically binding to BCMA are preferably derived from the same antibody and preferably identical in the CDR sequences, variable domain sequences VH and VL and the constant domain sequences CH1and CL. Preferably the amino acid sequences of the first and a second Fab fragment of an antibody specifically binding to BCMA are identical. Preferably the BCMA antibody is an antibody comprising the CDR sequences of antibody pSCHLI333, pSCHLI372 or pSCHLI373, an antibody comprising the VH sequences of antibody pSCHLI333, pSCHLI372 or pSCHLI373, or an antibody comprising the VH, and CH1, sequences of antibody pSCHLI333, pSCHLI372, pSCHLI373, or 83A10.

Preferably the bispecific antibody comprises as Fab fragments and Fc part, not more than one Fab fragment of an anti-CD3 antibody, not more than two Fab fragments of an anti-BCMA antibody and not more than one Fc part, preferably a human Fc part. Preferably the second Fab fragment of an anti-BCMA antibody is linked via its C-terminus either to the N-terminus of the Fab fragment of an anti-CD3 antibody or to the hinge region of the Fc part. Preferably linkage is performed between CH1 of BCMA-Fab and VH of CD3-Fab.

Such a preferred antibody according to the invention is the antibody characterized in comprising a heavy and light chain set of polypeptides
a) VH of SEQ ID NO:15, and VL of SEQ ID NO:2 (set pSCHLI333)
b) VH of SEQ ID NO:16, and VL of SEQ ID NO:2 (set pSCHLI372)
c) VH of SEQ ID NO:17, and VL of SEQ ID NO:2 (set pSCHLI373)
d) VH of SEQ ID NO:34, and VL of SEQ ID NO:2 (set 83A10)

Preferably the antibody according to the invention is further characterized in that it binds also specifically to cynomolgus BCMA.

Preferably the antibody portion specifically binding to human CD3, preferably the Fab fragment, is characterized in comprising a variable domain VH comprising the heavy chain CDRs of SEQ ID NO: 3, 4 and 5 as respectively heavy chain CDR1, CDR2 and CDR3 and a variable domain VL comprising the light chain CDRs of SEQ ID NO: 6, 7 and 8 as respectively light chain CDR1, CDR2 and CDR3 of the anti-CD3ε antibody (CDR MAB CD3 CH2527-VL7). Preferably the antibody portion specifically binding to human CD3 is characterized in that the variable domains are of SEQ ID NO:1 and 2 (VHVL MAB CD3 CH2527-VL7).

Preferably the antibody portion, preferably the Fab fragment, specifically binding to human BCMA is characterized in comprising a variable domain VH comprising the heavy chain CDRs CDR1H of SEQ ID NO:18, a CDR2H of SEQ ID NO:21, a CDR3H of SEQ ID NO: 24 and comprising a variable domain VL comprising the light chain CDRs CDR1L of SEQ ID NO:6, a CDR2L of SEQ ID NO:7, a CDR3L of SEQ ID NO: 8 (CDR MAB pSCHLI333). Preferably the antibody portion, preferably the Fab fragment, specifically binding to human BCMA is characterized in comprising a variable domain VH comprising the heavy chain CDRs CDR1H of SEQ ID NO:19, a CDR2H of SEQ ID NO:22, a CDR3H of SEQ ID NO: 25 and a variable domain VL comprising the light chain CDR1L of SEQ ID NO:6, a CDR2L of SEQ ID NO:7, a CDR3L of SEQ ID NO: 8 (CDR MAB pSCHLI372). Preferably the antibody portion, preferably the Fab fragment, specifically binding to human BCMA is characterized in comprising a variable domain VH comprising the heavy chain CDRs CDR1H of SEQ ID NO:20, a CDR2H of SEQ ID NO:23, a CDR3H of SEQ ID NO: 26 and a variable domain VL comprising the light chain CDR1L of SEQ ID NO:6, a CDR2L of SEQ ID NO:7, a CDR3L of SEQ ID NO: 8 (CDR MAB pSCHLI373). Preferably the antibody portion, preferably the Fab fragment, specifically binding to human BCMA is characterized in comprising a variable domain VH comprising the heavy chain CDRs CDR1H of SEQ ID NO:35, a CDR2H of SEQ ID NO:36, a CDR3H of SEQ ID NO: 37 and a variable domain VL comprising the light chain CDR1L of SEQ ID NO: 6, a CDR2L of SEQ ID NO: 7, a CDR3L of SEQ ID NO: 8 (CDR MAB 83A10).

Preferably the antibody portion, preferably the Fab fragment, specifically binding to human BCMA is characterized in comprising a VH of SEQ ID NO: 15. Preferably the antibody portion, preferably the Fab fragment, specifically binding to human BCMA is characterized in comprising a VH of SEQ ID NO:16 . Preferably the antibody portion, preferably the Fab fragment, specifically binding to human BCMA is characterized in comprising a VH of SEQ ID NO: 17. Preferably the antibody portion, preferably the Fab fragment, specifically binding to human BCMA is characterized in comprising a VH of SEQ ID NO:34.

The invention relates preferably to a bispecific antibody specifically binding to the extracellular domain of human BCMA and to human CD3ε, characterized in comprising a heavy and light chain set selected from the group consisting of polypeptides
i) SEQ ID NO:27, SEQ ID NO:28, and SEQ ID NO:29 (set 1),
ii) SEQ ID NO:29, SEQ ID NO:30, and SEQ ID NO:31 (set 2), and
iii) SEQ ID NO:29, SEQ ID NO:32, and SEQ ID NO:33 (set 3).

**Table 1: Antibody sequences of the anti BCMA antibodies**

| | SEQ ID NO: | | | |
|---|---|---|---|---|
| BCMA antibody | VH | CDR1H | CDR2H | CDR3H |
| pSCHLI333 | 15 | 18 | 21 | 24 |
| pSCHLI372 | 16 | 19 | 22 | 25 |
| pSCHLI373 | 17 | 20 | 23 | 26 |
| 83A10 | 34 | 35 | 36 | 37 |

**Table 2: Sequences of the building blocks**

| **Building block** | **SEQ ID NO:** |
|---|---|
| BCMA pSCHLI333 VH_CH1 x CD3 VH_CH1 Fc knob LALA PG | 27 |
| BCMA pSCHLI333 HC hole LALA PG | 28 |
| CD3 (BCMA) hum IgG1 LC (common light chain) | 29 |
| BCMA pSCHLI372 VH_CH1 x CD3 VH_CH1 Fc knob LALA PG | 30 |
| BCMA pSCHLI372 HC hole LALA PG | 31 |
| BCMA pSCHLI373 VH_CH1 x CD3 VH_CH1 Fc knob LALA PG | 32 |
| BCMA pSCHLI373 HC hole LALA PG | 33 |

**Table 3: Sequences of the anti-CD3 antibody**

| | **SEQ ID NO:** | | | | | | | |
|---|---|---|---|---|---|---|---|---|
| CH2527-VL7 | VH | VL | CDR1H | CDR2H | CDR3H | CDR1L | CDR2L | CDR3L |
| | 1 | 2 | 3 | 4 | 5 | 6 | 7 | 8 |

To make the following (2+1) Fc-containing anti-BCMA/anti-CD3 TCBs CLC, the respective "building blocks" / sequence IDs as mentioned in the table 2 above were needed:
pSCHLI333-TCB: 27, 28, 29 x3
pSCHLI372-TCB: 29 x3, 30, 31
pSCHLI373-TCB: 29 x3, 32, 33

Preferably an antibody according to the invention shows high affinity to BCMA and low aggregation.

The invention further relates to a nucleic acid set encoding a respective heavy and light chain set.

Preferably the bispecific antibody according to the invention comprising constant heavy regions CH2/CH3 of IgG1 subclass is characterized in comprising the mutations L234A, L235A and P239G (numbering according to Kabat) to avoid FcR and C1q binding and minimizing ADCC/CDC. The advantage is that such an antibody of the invention mediates its tumor cell killing efficacy purely by the powerful mechanism of T-cell redirection/activation. Additional mechanisms of action like effects on complement system and on effector cells expressing FcR are avoided and the risk of side-effects is decreased.

Preferably an antibody according to the invention is characterized by showing tumor growth inhibition of more than 70%, preferably of more than 85%, preferably of close to 100% in a multiple myeloma xenograft model (e.g. xenograft with NCI-H929 cells or RPMI8226 cells or U266B1 cells or L-363 cells) at a dose of 1 mg/kg body weight (BW) administered intravenously (i.v.) or subcutaneously (s.c.) or intraperitoneal (i.p.) twice a week or once a week, preferably 0.5 mg/kg BW administered i.v. or i.p. or s.c. twice a week or once a week, preferably at 0.1 mg/kg BW administered i.v. or i.p. or s.c. twice a week or once a week, preferably at 0.05 mg/kg BW administered i.v. or i.p. or s.c. twice a week or once a week, preferably at 0.01 mg/kg BW administered i.v. or i.p. or s.c twice a week or once a week, preferably at 5µg/kg BW administered i.v. or i.p. or s.c. twice a week or once a week.

Preferably an antibody according to the invention is characterized by an elimination half-life in mice, preferably cynomolgus monkeys of longer than 24 hours, preferably 3 days or longer, preferably half-life is measured for the doses which are effective in the xenograft model at twice or once a week administration.

Bispecific antibodies binding to a target on tumor cells and to CD3 and having the molecular format (scFV)₂ have very short elimination half-life of 1 to 4 hours. In the clinical trials with the (scFV)₂ bispecific CD19xCD3 antibody blinatumomab, this compound had to be administered via a pump carried by the patients over weeks and months (Topp et al. J Clin Oncol 2011; 29(18): 2493-8). Compared to a twice a week or once a week iv or sc administration, treatment administered via a pump is much less convenient for the patients and also much more risky (e.g. failure of pump, issues with the catheter).

Preferably an antibody according to the invention is characterized in showing an EC50 value for binding to NCI-H929 cells (ATCC® CRL-9068™) of 500 nM or lower, preferably an EC50 value of 350 nM or lower, preferably an EC50 value of 100 nM and lower.

Preferably an antibody according to the invention is characterized by its capability to induce redirected killing of NCI-H929 tumor cells in the presence of human T cells with an EC50 lower than 1 nM, preferably 0.5 nM, preferably 0.1 nM and lower.

Stability of bispecific antibodies can be affected in practical conditions and clinical applications. Despite recent antibody engineering improvements, some recombinant proteins and molecular formats (e.g. scFVs fragments) tend to denature and form aggregates more easily than other under stress conditions (Worn and Pluckthun. J Mol Biol 2001; 305, 989-1010). Preferably an antibody according to this invention is characterized in that said antibody stored in standard formulation buffer at 37°C preferably at 40°C, for 10 days, preferably up to 2 weeks, preferably up to 4 weeks, does not result in more than 10% changes (Δ), preferably not more than 5% changes (Δ), in high molecular weight (HMW) species and/or low molecular weight (LMW) species and/or monomer content as compared to the said antibody stored in the same formulation buffer at -80°C for the same period of storage.

Preferably a bispecific antibody according to the invention is characterized by its capability to induce redirected killing of multiple myeloma patient primary myeloma cells in the presence of human T cells. Preferably the bispecific antibody according to the invention is characterized in that the CH3 domain of one heavy chain and the CH3 domain of the other heavy chain each meet at an interface which comprises an original interface between the antibody CH3 domains; wherein said interface is altered to promote the formation of the bispecific antibody, wherein the alteration is characterized in that:
a) the CH3 domain of one heavy chain is altered, so that within the original interface the CH3 domain of one heavy chain that meets the original interface of the CH3 domain of the other heavy chain within the bispecific antibody, an amino acid residue is replaced with an amino acid residue having a larger side chain volume, thereby generating a protuberance within the interface of the CH3 domain of one heavy chain which is positionable in a cavity within the interface of the CH3 domain of the other heavy chain and
b) the CH3 domain of the other heavy chain is altered, so that within the original interface of the second CH3 domain that meets the original interface of the first CH3 domain within the bispecific antibody an amino acid residue is replaced with an amino acid residue having a smaller side chain volume, thereby generating a cavity within the interface of the second CH3 domain within which a protuberance within the interface of the first CH3 domain is positionable.

Preferably the antibody according to the invention is characterized in that said amino acid residue having a larger side chain volume is selected from the group consisting of arginine (R), phenylalanine (F), tyrosine (Y), tryptophan (W). Preferably the antibody according to the invention is characterized in that said amino acid residue having a smaller side chain volume is selected from the group consisting of alanine (A), serine (S), threonine (T), valine (V). Preferably the antibody according to the invention is characterized in that both CH3 domains are further altered by the introduction of cysteine (C) as amino acid in the corresponding positions of each CH3 domain. Preferably the antibody according to the invention is characterized in that one of the constant heavy chain domains CH3 of both heavy chains is replaced by a constant heavy chain domain CH1; and the other constant heavy chain domain CH3 is replaced by a constant light chain domain CL.

Preferably the antibody according to the invention is further characterized in that it binds also specifically to cynomolgus BCMA.

A further embodiment of the invention is one or more nucleic acids encoding the amino acid sequences of an antibody according to the invention, preferably of the building blocks according to the invention.

Further embodiments of the invention are expression vectors comprising nucleic acids according to the invention capable of expressing said nucleic acid in a host cell.

A further embodiment of the invention is a method for the preparation of a bispecific antibody according to the invention comprising the steps of
a) transforming a host cell with vectors comprising nucleic acid molecules encoding the light chains and heavy chains of an antibody according to this invention
b) culturing the host cell under conditions that allow synthesis of said antibody molecule, and
c) recovering said antibody molecule from said culture.

A further embodiment of the invention is a host cell comprising vectors comprising nucleic acid molecules encoding the light chains and heavy chains of an antibody according to the invention.

A further preferred embodiment of the invention is a pharmaceutical composition comprising an antibody according to the invention and a pharmaceutically acceptable excipient.

A further embodiment of the invention is a diagnostic composition comprising an antibody according to the invention.

A further embodiment of the invention is a method for the treatment of a patient in need of therapy, characterized by administering to the patient a therapeutically effective amount of a bispecific antibody according to the invention.

A further preferred embodiment of the invention is a pharmaceutical composition comprising an antibody according to the invention for use as a medicament. A further preferred embodiment of the invention is a pharmaceutical composition comprising an antibody according to the invention for use as a medicament in the treatment of plasma cell disorders. A further preferred embodiment of the invention is a pharmaceutical composition comprising an antibody according to the invention for use as a medicament in the treatment of multiple myeloma. A further embodiment of the invention is an antibody according to the invention for the treatment of plasma cell disorders like multiple myeloma or other B-cell disorders expressing BCMA. Multiple myeloma is a B-cell malignancy characterized by a monoclonal expansion and accumulation of abnormal plasma cells in the bone marrow compartment. Multiple myeloma also involves circulating clonal B cells with same IgG gene rearrangement and somatic hypermutation. Multiple myeloma arises from an asymptomatic, premalignant condition called monoclonal gammopathy of unknown significance (MGUS), characterized by low levels of bone marrow plasma cells and a monoclonal protein. Multiple myeloma cells proliferate at low rate. Multiple myeloma results from a progressive occurrence of multiple structural chromosomal changes (e.g. unbalanced translocations). Multiple myeloma involves the mutual interaction of malignant plasma cells and bone marrow microenvironment (e.g. normal bone marrow stromal cells). Clinical signs of active Multiple myeloma include monoclonal antibody spike, plasma cells overcrowding the bone marrow, lytic bone lesions and bone destruction resulting from overstimulation of osteoclasts (Dimopulos & Terpos, Ann Oncol 2010; 21 suppl 7: vii143-150). Another B-cell disorder involving plasma cells i.e. expressing BCMA is systemic lupus erythematosus (SLE), also known as lupus. SLE is a systemic, autoinmune disease that can affect any part of the body and is represented with the immune system attacking the body's own cells and tissue, resulting in chronic inflammation and tissue damage. It is a Type III hypersensitivity reaction in which antibody-immune complexes precipitate and cause a further immune response (Inaki & Lee, Nat Rev Rheumatol 2010; 6: 326-337). A further preferred embodiment of the invention is pharmaceutical composition comprising an antibody according to the invention for use as a medicament in the treatment of systemic lupus erythematosus.

### Description of the Figures

Figure 1. Bispecific bivalent and trivalent antibodies comprising only the Fab fragments (specific to CD3 and BCMA) with or without an Fc part as specified: (A) Fab BCMA-Fc-Fab CD3; (B) Fab BCMA-Fc-Fab CD3-Fab BCMA; (C) Fab BCMA-Fc-Fab BCMA-Fab CD3; (D) Fc-Fab CD3-Fab BCMA; (E) Fc-Fab BCMA-Fab CD3; (F) Fab CD3-Fab BCMA-Fab BCMA; (G) Fab CD3-Fab BCMA. Preferably, the LC of Fab CD3 and Fab BCMA are identical (common LC) to avoid LC mispairing and reduce side-products. Fab CD3 and Fab BCMA are linked to each other with flexible linkers.

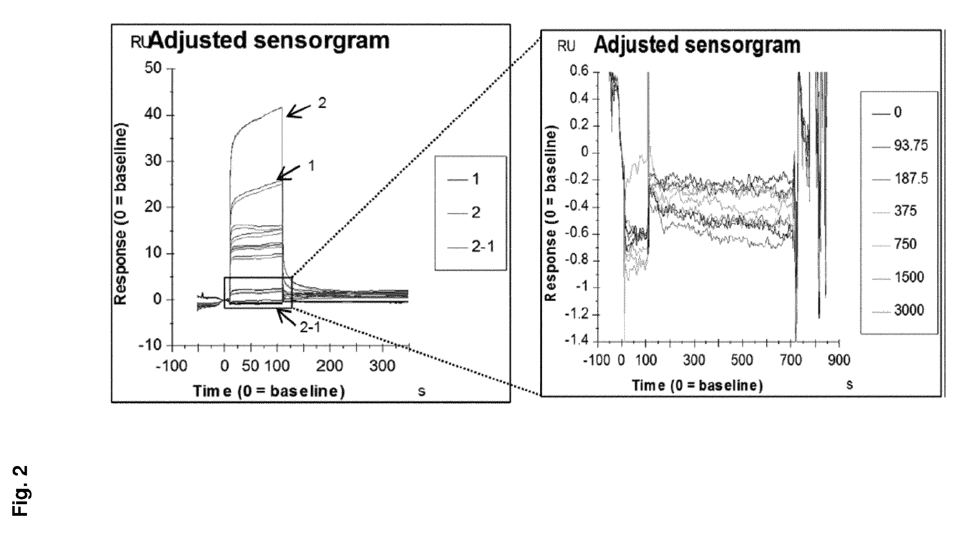

Figure 2. Lack of binding of BCMA IgG antibody to TACI receptor as detected by surface plasmon resonance (SPR). Curve 1 corresponds to the signal on reference channel, curve 2 to the channel where the binding occurs (binding channel) and the curve 2-1 is the subtracted signal (binding channel - reference channel), meaning that this is the signal due to the binding event. SPR binding assay clearly demonstrated that pSCHLI372 IgG did not bind to human TACI receptor (see Example 4).

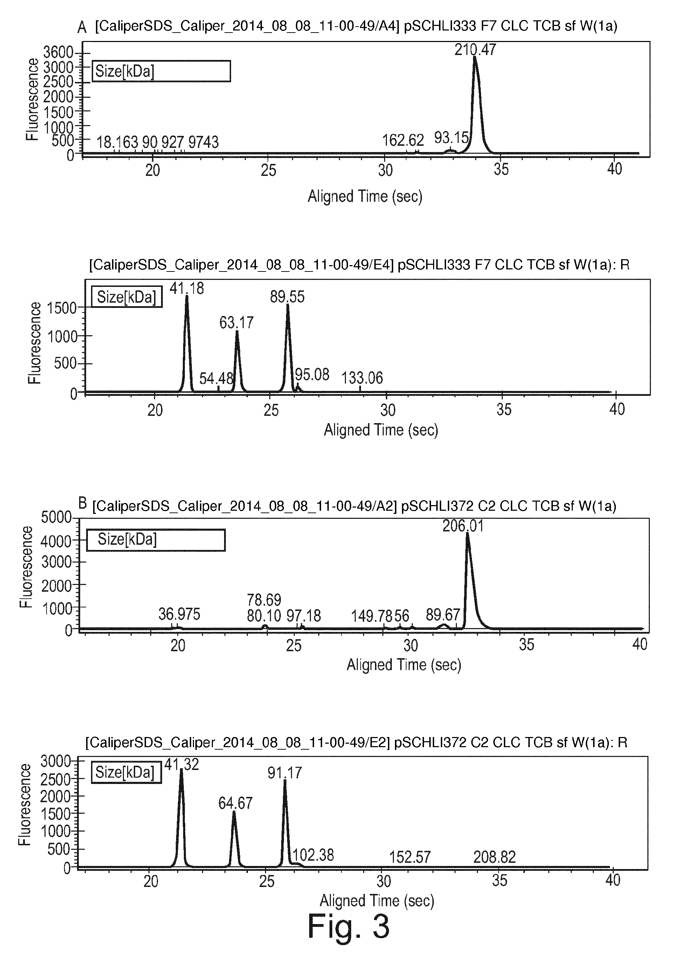

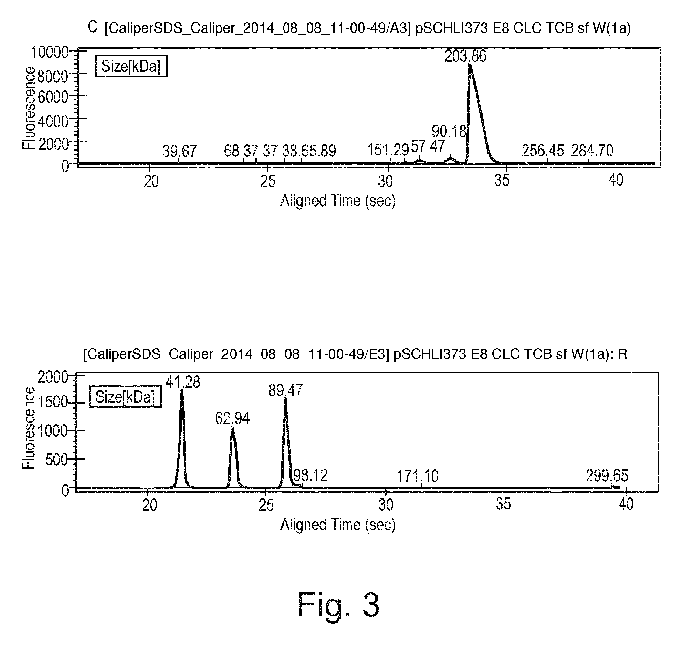

Figure 3. Production and purification of BCMA-TCB CLC. CE-SDS graphs (non-reduced (top) and reduced (bottom)) of the final protein preparations after Protein A (PA) affinity chromatography and size exclusion chromatographic (SEC) purification steps applied to (A) pSCHLI333-TCB CLC, (B) pSCHLI372-TCB CLC, (C) pSCHLI373-TCB CLC. All three molecules are of molecular format as described in Figure 1B (see Example 6).

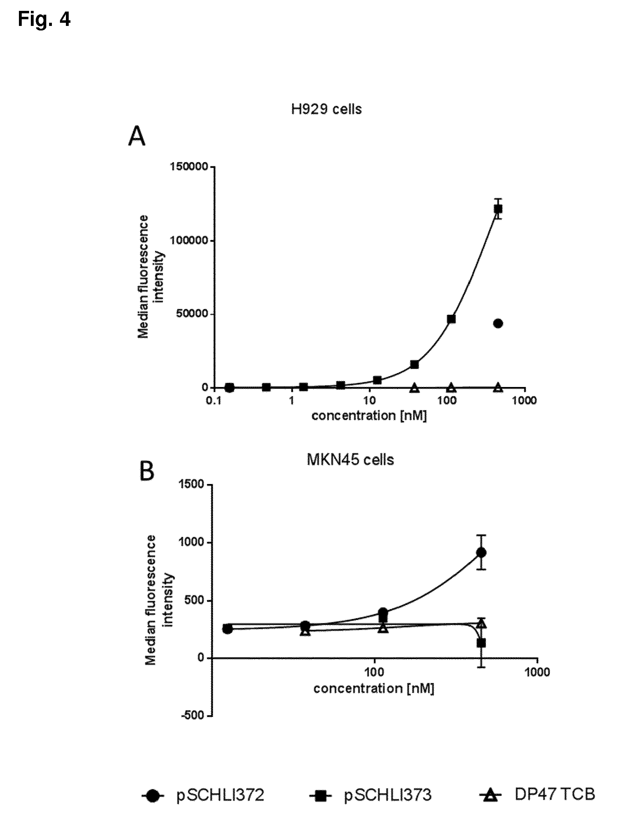

Figure 4. Binding of BCMA-TCB CLC antibodies on BCMA^{hi}-positive H929 cells by flow cytometry. The median fluorescence intensity of BCMA-TCB CLC antibodies were plotted in function of antibody concentrations (0.12 to 500 nM); (A) pSCHLI372-TCB CLC and pSCHLI373-TCB CLC on H929 cells (A) and MKN45 cells (B). DP47-TCB is a negative control TCB which did not bind to BCMA at concentrations below 100 nM (see Example 7).

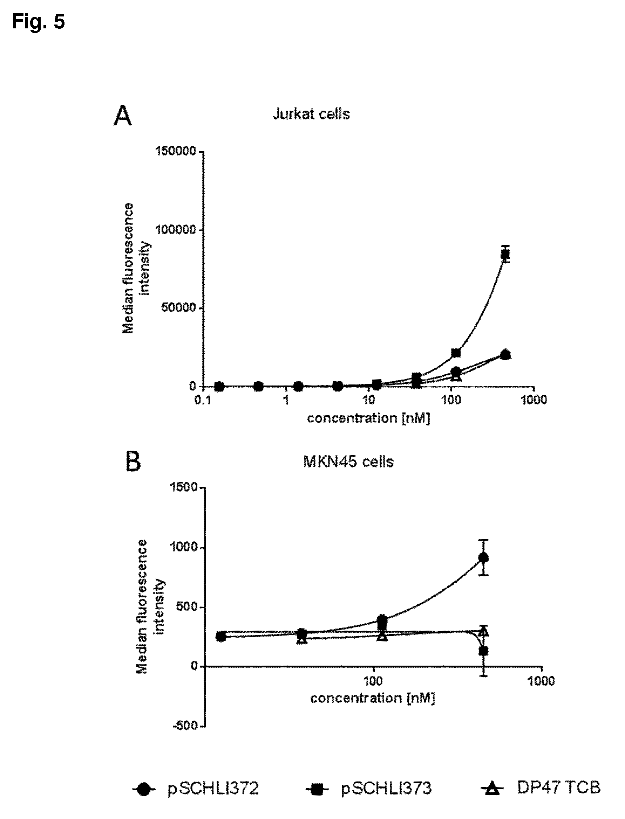

Figure 5. Binding of BCMA-TCB CLC antibodies on CD3-positive Jurkat T cells as measured by flow cytometry. Median fluorescence intensity for BCMA-TCB CLC antibodies (pSCHLI372-TCB CLC and pSCHLI373-TCB CLC) binding to Jurkat T cells and plotted in function of antibody concentration. Non-binding to BCMA-negative and CD3-negative MKN45 cells at concentrations below 100 nM (see Example 8).

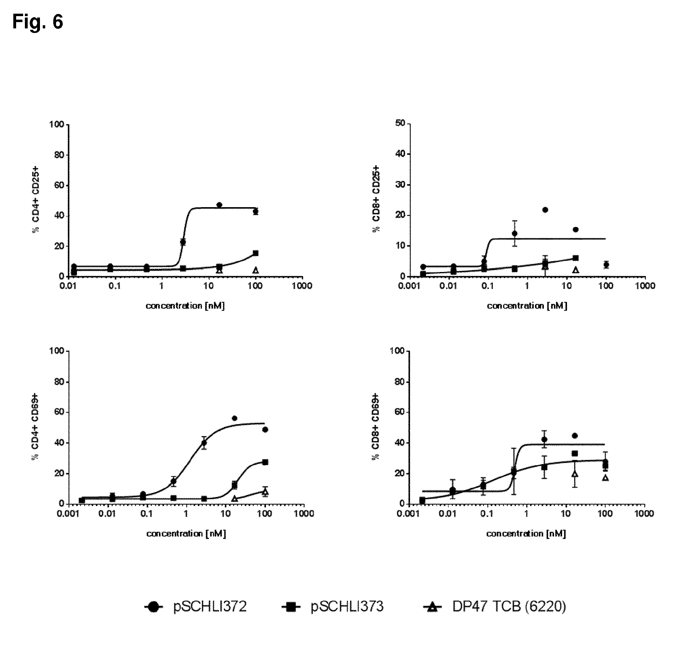

Figure 6. T-cell activation mediated by BCMA-TCB CLC antibodies in presence of H929 cells as detected by flow cytometry. Expression level of the early activation marker CD69 and the late activation marker CD25 on CD4⁺ and CD8⁺ T cells after 48 hours of incubation. pSCHLI372-TCB CLC and pSCHLI373-TCB CLC antibodies induced an up-regulation of CD69 and CD25 activation markers in a concentration-dependent and specific manner in the presence of BCMA-positive target cells. E:T ratio used as 10 PBMCs:1 H929 cell; cells were incubated for 48h before measurement of CD69 and CD25 upregulation. DP47-TCB which is a negative control TCB did not induce T-cell activation. Representative results from two independent experiments (see Example 9).

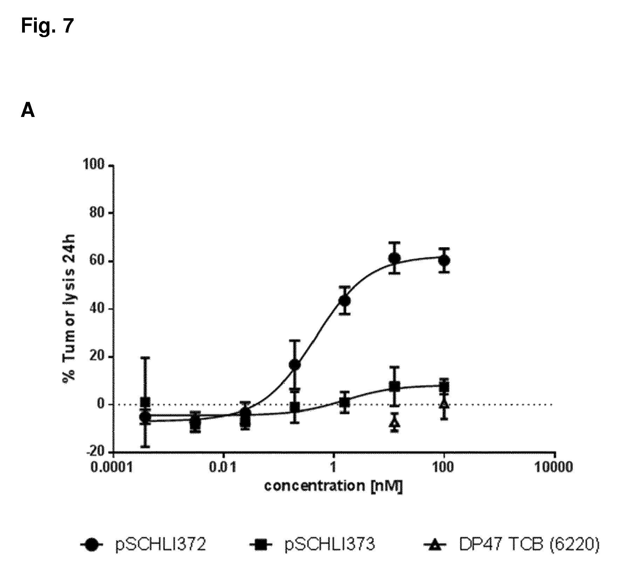

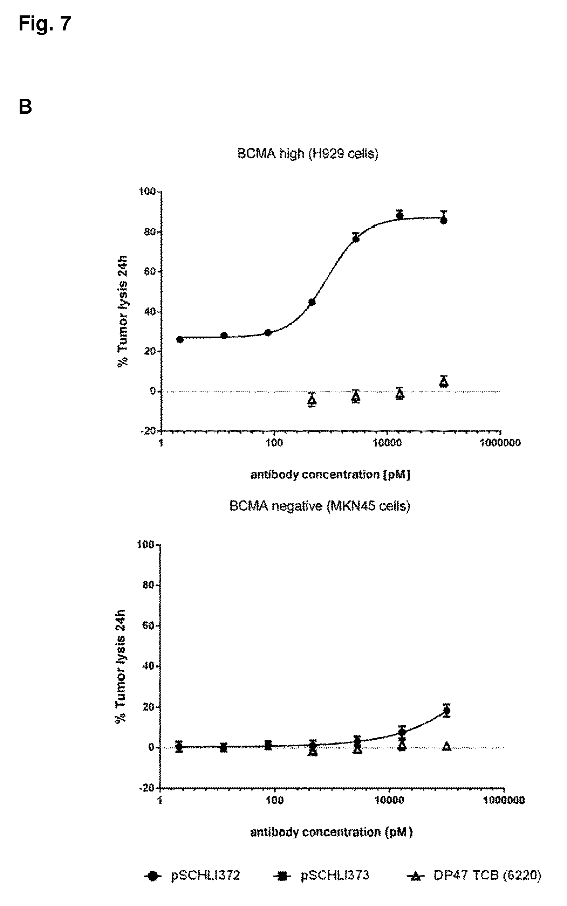

Figure 7. BCMA-TCB CLC antibodies induce T-cell redirected killing of BCMA^{hi}-positive H929 myeloma cells as detected by colorimetric LDH release assay. BCMA-TCB CLC antibodies pSCHLI372-TCB CLC (A, B) and pSCHLI373-TCB CLC (A) induced a concentration-dependent killing of BCMA^{hi}-positive H929 myeloma cells as measured by LDH release. DP47-TCB which is a negative control TCB that does not bind to BCMA but only to CD3 did not induce H929 cell killing. E:T ratio used as 10 PBMCs:1 H929 cell; cells were incubated for 24h before measurement of LDH release. Representative results from three independent experiments (see Example 10).

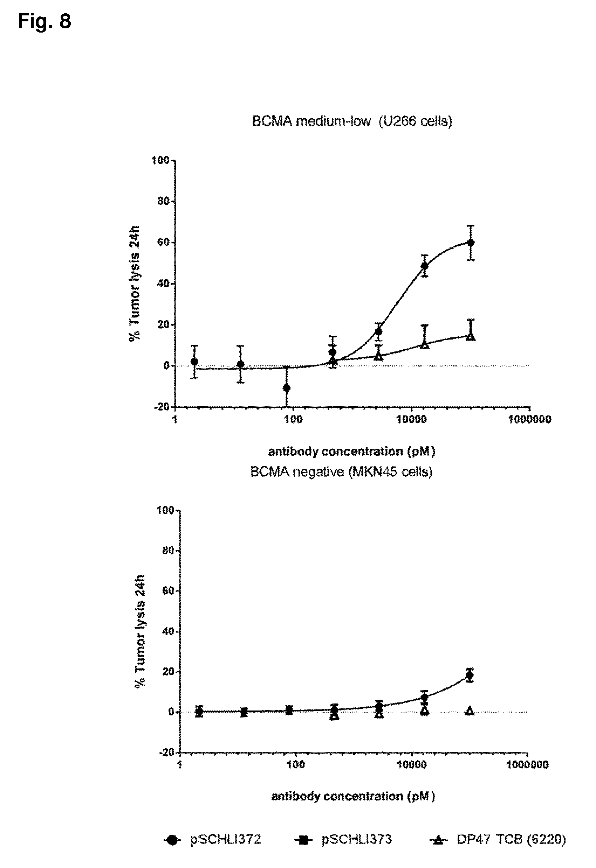

Figure 8. BCMA-TCB CLC antibodies induce T-cell redirected killing of BCMA^{med/lo}-positive U266 myeloma cells as detected by colorimetric LDH release assay. BCMA-TCB CLC antibodies pSCHLI372-TCB CLC and pSCHLI373-TCB CLC induced a concentration-dependent killing of BCMA^{med/lo}-positive U266 myeloma cells as measured by LDH release. DP47-TCB which is a negative control TCB that does not bind to BCMA but only to CD3 did not induce H929 cell killing. E:T ratio used as 10 PBMCs:1 U266 cell; cells were incubated for 24h before measurement of LDH release. Representative results from two independent experiments (see Example 11).

### Detailed Description of the Invention

The term "target" as used herein means either BCMA or CD3.The term "first target and second target" means either CD3 as first target and BCMA as second target or means BCMA as first target and CD3 as second target.

The term "BCMA" as used herein relates to human B cell maturation target, also known as BCMA; TR17_HUMAN, TNFRSF17 (UniProt Q02223), which is a member of the tumor necrosis receptor superfamily that is preferentially expressed in differentiated plasma cells. The extracellular domain of BCMA consists according to UniProt of amino acids 1 - 54 (or 5-51). The term "antibody against BCMA, anti BCMA antibody" as used herein relates to an antibody specifically binding to BCMA. The term "CD3ε or CD3" as used herein relates to human CD3ε described under UniProt P07766 (CD3E_HUMAN). The term "antibody against CD3, anti CD3 antibody" relates to an antibody binding to CD3ε. Preferably the antibody comprises a variable domain VH comprising the heavy chain CDRs of SEQ ID NO: 3, 4 and 5 as respectively heavy chain CDR1, CDR2 and CDR3 and a variable domain VL comprising the light chain CDRs of SEQ ID NO: 6, 7 and 8 as respectively light chain CDR1, CDR2 and CDR3. Preferably the antibody comprises the variable domains of SEQ ID NO:1 (VH) and SEQ ID NO:2 (VL). The term "antibody against CD3, anti CD3 antibody" as used herein relates to an antibody specifically binding to CD3.

"Specifically binding to CD3 or BCMA" refer to an antibody that is capable of binding CD3 or BCMA (the targets) with sufficient affinity such that the antibody is useful as a therapeutic agent in targeting CD3 or BCMA. In some embodiments, the extent of binding of an anti-CD3 or BCMA antibody to an unrelated, non-CD3 or non-BCMA protein is about 10-fold preferably >100-fold less than the binding of the antibody to CD3 or BCMA as measured, e.g., by surface plasmon resonance (SPR) e.g. Biacore®, enzyme-linked immunosorbent (ELISA) or flow cytometry (FACS). Preferably the antibody that binds to CD3 or BCMA has a dissociation constant (Kd) of 10⁻⁸ M or less, preferably from 10⁻⁸ M to 10⁻¹³ M, preferably from 10⁻⁹ M to 10⁻¹³ M. Preferably the anti-CD3 and/or anti-BCMA antibody binds to an epitope of CD3 and/or BCMA that is conserved among CD3 and/or BCMA from different species, preferably among human and cynomolgus. "Bispecific antibody specifically binding to CD3 and BCMA" or "antibody according to the invention" refers to a respective definition for binding to both targets. An antibody specifically binding to BCMA (or BCMA and CD3) does not bind to other human antigens. Therefore in an ELISA, OD values for such unrelated targets will be equal or lower to that of the limit of detection of the specific assay, preferably > 0.3 ng/mL, or equal or lower to OD values of control samples without plate-bound-BCMA or with untransfected HEK293 cells.

The term "CD3ε or CD3 binding part" as used herein relates to the combination of an antibody heavy chain consisting of VH and CH1 and an antibody light chain consisting of VL and CL as enclosed in a Fab fragment of an antibody specifically binding to CD3. In a bispecific antibody according to the invention a CD3 binding part, "the CD3 binding portion", comprising as light chain said common light chain, is preferably incorporated once.

The term "BCMA binding part" as used herein relates to the combination of an antibody heavy chain consisting of VH and CH1 and an antibody light chain consisting of VL and CL as enclosed in a Fab fragment of an antibody specifically binding to BCMA. In a bispecific antibody according to the invention a BCMA binding part, "the BCMA binding portion", comprising as light chain said common light chain, is preferably incorporated once or twice.

Preferably an antibody according to the invention is characterized in showing an EC50 value for binding to NCI-H929 cells (ATCC® CRL-9068™) of 500 nM or lower, preferably an EC50 value of 350 nM and lower, preferably an EC50 value of 100 nM and lower.

Preferably, an antibody according to this invention is characterized by its capability to bind to U266 (ATCC® TIB-196^{™}) cells.

In one preferred embodiment, an antibody according to the invention is characterized by its capability to bind to human T cells.

Preferably, an antibody according to this invention is characterized by its capability to bind to cynomolgus monkey BCMA transiently expressed on HEK-cells.

In a preferred embodiment, an antibody according to this invention is characterized by its capability to induce CD4⁺ and CD8⁺ T-cell activation in the presence of tumor cells expressing BCMA.

Preferably an antibody according to the invention is characterized by its capability to induce redirected killing of NCI-H929 tumor cells in the presence of human T cells with an EC50 lower than 1 nM, preferably 0.5 nM, preferably 0.1 nM and lower.

The term "antibody" as used herein refers to a monoclonal antibody. An antibody consists of two pairs of a "light chain" (LC) and a "heavy chain" (HC) (such light chain (LC) /heavy chain pairs are abbreviated herein as LC/HC). The light chains and heavy chains of such antibodies are polypeptides consisting of several domains. Each heavy chain comprises a heavy chain variable region (abbreviated herein as HCVR or VH) and a heavy chain constant region. The heavy chain constant region comprises the heavy chain constant domains CH1, CH2 and CH3 (antibody classes IgA, IgD, and IgG) and optionally the heavy chain constant domain CH4 (antibody classes IgE and IgM). Each light chain comprises a light chain variable domain VL and a light chain constant domain CL. The variable domains VH and VL can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The "constant domains" of the heavy chain and of the light chain are not involved directly in binding of an antibody to a target, but exhibit various effector functions.

The "light chain of an antibody" as used herein is a polypeptide comprising in N-terminal to C-terminal direction a light chain variable domain (VL), and a light chain constant domain (CL), abbreviated as VL-CL. "The "heavy chain of an antibody" as used herein is a polypeptide comprising in N-terminal to C-terminal direction a heavy chain variable domain (VH) and a constant heavy chain domain 1 (CH1).

There exist several approaches for CH3-modifications to enforce the heterodimerization, which are well described e.g. in WO96/27011, WO98/050431, EP1870459, WO2007/110205, WO2007/147901, WO2009/089004, WO2010/129304, WO2011/90754, WO2011/143545, WO2012058768, WO2013157954, WO2013096291. Typically in all such approaches the first CH3 domain and the second CH3 domains are both engineered in a complementary manner so that each CH3 domain (or the heavy chain comprising it) cannot longer homodimerize with itself but is forced to heterodimerize with the complementary engineered other CH3 domain ( so that the first and second CH3 domain heterodimerize and no homodimers between the two first or the two second CH3 domains are formed). In one preferred embodiment of the invention (in case the antibody according to the invention comprises CH3 domains in the heavy chains) the CH3 domains of said multispecific antibody according to the invention can be altered by the "knob-into-holes" technology which is described in detail with several examples in e.g. WO 96/027011, Ridgway, J.B., et al., Protein Eng. 9 (1996) 617-621; and Merchant, A.M., et al., Nat. Biotechnol. 16 (1998) 677-681; WO98/ 050431. In this method the interaction surfaces of the two CH3 domains are altered to increase the heterodimerisation of both heavy chains containing these two CH3 domains. Each of the two CH3 domains (of the two heavy chains) can be the "knob", while the other is the "hole".

Thus in one embodiment of the invention said antibody according to the invention (comprises a CH3 domain in each heavy chain and) is further characterized in that the first CH3 domain of the first heavy chain of the antibody under a) and the second CH3 domain of the second heavy chain of the antibody under b) each meet at an interface which comprises an original interface between the antibody CH3 domains, wherein said interface is altered to promote the formation of the antibody according to the invention, wherein the alteration is characterized in that:
i) the CH3 domain of one heavy chain is altered, so that within the original interface of the CH3 domain of one heavy chain that meets the original interface of the CH3 domain of the other heavy chain within the antibody according to the invention, an amino acid residue is replaced with an amino acid residue having a larger side chain volume, thereby generating a protuberance within the interface of the CH3 domain of one heavy chain which is positionable in a cavity within the interface of the CH3 domain of the other heavy chain and
ii) the CH3 domain of the other heavy chain is altered, so that within the original interface of the second CH3 domain that meets the original interface of the first CH3 domain within the antibody according to the invention an amino acid residue is replaced with an amino acid residue having a smaller side chain volume, thereby generating a cavity within the interface of the second CH3 domain within which a protuberance within the interface of the first CH3 domain is positionable.

Preferably said amino acid residue having a larger side chain volume is selected from the group consisting of arginine (R), phenylalanine (F), tyrosine (Y), tryptophan (W).

In one aspect of the invention both CH3 domains are further altered by the introduction of cysteine (C) as amino acid in the corresponding positions of each CH3 domain such that a disulfide bridge between both CH3 domains can be formed.

Other techniques for CH3-modifications to enforcing the heterodimerization are contemplated as alternatives of the invention and described e.g. in WO96/27011, WO98/050431, EP1870459, WO2007/110205, WO2007/147901, WO2009/089004, WO2010/129304, WO2011/90754, WO2011/143545, WO2012/058768, WO2013/157954, WO2013/157953, WO2013/096291.

In one embodiment the antibody according to the invention is of IgG2 isotype and the heterodimerization approach described in WO2010/129304 can be used alternatively.

The term "antibody" includes e.g. mouse antibodies, human antibodies, chimeric antibodies, humanized antibodies and genetically engineered antibodies (variant or mutant antibodies) as long as their characteristic properties are retained. Especially preferred are human or humanized antibodies, especially as recombinant human or humanized antibodies. The terms "monoclonal antibody" or "monoclonal antibody composition" as used herein refer to a preparation of antibody molecules of a single amino acid composition.

The terms "bispecific antibody" and "antibody according to the invention" as used herein refer to an antibody in which one of the two pairs of heavy chain and light chain (HC/LC) is specifically binding to BCMA and the other one is specifically binding to CD3 or preferably to CD3 and BCMA. The term "valent" as used within the current application denotes the presence of a specified number of binding sites in an antibody molecule. A bivalent antibody according to this invention has two binding sites, one for CD3 and the other for BCMA. As such, the term "trivalent", denote the presence of three binding sites in an antibody according to the invention, which are two binding sites for BCMA and one binding site for CD3.

There are five types of mammalian antibody heavy chains denoted by the Greek letters: α, δ ε, γ, and µ (Janeway CA, Jr et al (2001). Immunobiology. 5th ed., Garland Publishing). The type of heavy chain present defines the class of antibody; these chains are found in IgA, IgD, IgE, IgG, and IgM antibodies, respectively (Rhoades RA, Pflanzer RG (2002). Human Physiology, 4th ed., Thomson Learning). Distinct heavy chains differ in size and composition; α and γ contain approximately 450 amino acids, while µ and ε have approximately 550 amino acids. Each heavy chain has two regions, the constant region and the variable region. The constant region is identical in all antibodies of the same isotype, but differs in antibodies of different isotype. Heavy chains γ, α and δ have a constant region composed of three constant domains CH1, CH2, and CH3 (in a line), and a hinge region for added flexibility (Woof J, Burton D Nat Rev Immunol 4 (2004) 89-99); heavy chains µ and ε have a constant region composed of four constant domains CH1, CH2, CH3, and CH4 (Janeway CA, Jr et al (2001). Immunobiology. 5th ed., Garland Publishing). The variable region of the heavy chain differs in antibodies produced by different B cells, but is the same for all antibodies produced by a single B cell or B cell clone. The variable region of each heavy chain is approximately 110 amino acids long and is composed of a single antibody domain.

A "common light chain" as used herein is the only light chain used in a bispecific antibody according to the invention. In mammals there are two types of light chain, which are called lambda (λ) and kappa (κ). A light chain has two successive domains: one constant domain CL and one variable domain VL. The approximate length of a light chain is 211 to 217 amino acids. Preferably the light chain is a kappa (κ) light chain, and the constant domain CL is preferably derived from a kappa (K) light chain (the constant domain CK). Preferably the heavy and light chain constant domains of the antibody according to the invention are human domains.

The "antibodies" according to the invention can be of any class (e.g. IgA, IgD, IgE, IgG, and IgM, preferably IgG or IgE), or subclass (e.g., IgG1, IgG2, IgG3, IgG4, IgA1 and IgA2, preferably IgG1), whereby both antibodies, from which the bivalent bispecific antibody according to the invention is derived, have an Fc part of the same subclass( e.g. IgG1, IgG4 and the like, preferably IgG1), preferably of the same allotype (e.g. Caucasian).

A "Fab fragment of an antibody" as used herein is a fragment on an antibody that binds to antigens. A Fab fragment of an antibody consists of two pairs of domains. In a wild-type antibody it is composed of one constant and one variable domain of each of the heavy chain (CH1 and VH) and the light chain (CL and VL). In a wild-type antibody and according to the invention the domain of the heavy and light chain domain pairs of a Fab fragment are not chemically linked together and are therefore not scFvs (single chain variable fragments).

A "Fc part of an antibody" is a term well known to the skilled artisan and defined on the basis of papain cleavage of antibodies. The antibodies according to the invention contain as Fc part, preferably a Fc part derived from human origin and preferably all other parts of the human constant regions. The Fc part of an antibody is directly involved in complement activation, C1q binding, C3 activation and Fc receptor binding. While the influence of an antibody on the complement system is dependent on certain conditions, binding to C1q is caused by defined binding sites in the Fc part. Such binding sites are known in the state of the art and described e.g. by Lukas, TJ., et al., J. Immunol. 127 (1981) 2555-2560; Brunhouse, R., and Cebra, J.J., MoI. Immunol. 16 (1979) 907-917; Burton, D.R., et al., Nature 288 (1980) 338-344; Thommesen, J.E., et al., MoI. Immunol. 37 (2000) 995-1004; Idusogie, E.E., et al., J. Immunol. 164 (2000) 4178-4184; Hezareh, M., et al., J. Virol. 75 (2001) 12161-12168; Morgan, A., et al., Immunology 86 (1995) 319-324; and EP 0 307 434. Such binding sites are e.g. L234, L235, D270, N297, E318, K320, K322, P331 and P329 (numbering according to EU index of Kabat, see below). Antibodies of subclass IgG1, IgG2 and IgG3 usually show complement activation, C1q binding and C3 activation, whereas IgG4 do not activate the complement system, do not bind C1q and do not activate C3. Preferably the Fc part is a human Fc part. Preferably the Fc part is a human IgGlFc part. Preferably the antibody according to the invention comprises in the human IgG1 Fc part amino acid substitution of Pro329 with glycine or arginine and/or substitutions L234A and L235A, preferably Pro329 with glycine and substitutions L234A and L235A.

Preferably the antibody according to the invention comprises as Fc part an Fc variant of a wild-type human IgG Fc region, said Fc variant comprising an amino acid substitution at position Pro329 and at least one further amino acid substitution, wherein the residues are numbered according to the EU index of Kabat, and wherein said antibody exhibits a reduced affinity to the human FcγRIIIA and/or FcγRIIA and /or FcγRI compared to an antibody comprising the wildtype IgG Fc region, and wherein the ADCC induced by said antibody is reduced to at least 20% of the ADCC induced by the antibody comprising a wild-type human IgG Fc region. In a specific embodiment Pro329 of a wild-type human Fc region in the antibody according to the invention is substituted with glycine or arginine or an amino acid residue large enough to destroy the proline sandwich within the Fc/Fcγ receptor interface, that is formed between the proline329 of the Fc and tryptophane residues Trp 87 and Tip 110 of FcγRIII (Sondermann et al.: Nature 406, 267-273 (20 July 2000)). In a further aspect of the invention the at least one further amino acid substitution in the Fc variant is S228P, E233P, L234A, L235A, L235E, N297A, N297D, or P331S and still in another embodiment said at least one further amino acid substitution is L234A (denotes that leucine 234 is substituted by alanine) and L235A of the human IgG1 Fc region or S228P and L235E of the human IgG4 Fc region. Such Fc variants are described in detail in WO2012130831.

The term "chimeric antibody" refers to an antibody comprising a variable region, i.e., binding region, from one source or species and at least a portion of a constant region derived from a different source or species, usually prepared by recombinant DNA techniques. Chimeric antibodies comprising a murine variable region and a human constant region are preferred. Other preferred forms of "chimeric antibodies" encompassed by the present invention are those in which the constant region has been modified or changed from that of the original antibody to generate the properties according to the invention, especially in regard to C1q binding and/or Fc receptor (FcR) binding. Such chimeric antibodies are also referred to as "class-switched antibodies". Chimeric antibodies are the product of expressed immunoglobulin genes comprising DNA segments encoding immunoglobulin variable regions and DNA segments encoding immunoglobulin constant regions. Methods for producing chimeric antibodies involve conventional recombinant DNA and gene transfection techniques are well known in the art. See, e.g., Morrison, S.L., et al., Proc. Natl. Acad. Sci. USA 81 (1984) 6851-6855; US Patent Nos. 5,202,238 and 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Particularly preferred CDRs correspond to those representing sequences recognizing the targets noted above for chimeric antibodies. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

The term "human antibody", as used herein, is intended to include antibodies having variable and constant regions derived from human germ line immunoglobulin sequences. Human antibodies are well-known in the state of the art (van Dijk, M.A., and van de Winkel, J.G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon target challenge (see, e.g., Jakobovits, A., et al., Proc. Natl. Acad. Sci. USA 90 (1993) 2551-2555; Jakobovits, A., et al., Nature 362 (1993) 255-258; Bruggemann, M., et al., Year Immunol. 7 (1993) 33-40). Human antibodies can also be produced in phage display libraries (Hoogenboom, H.R., and Winter, G., J. MoI. Biol. 227 (1992) 381-388; Marks, J.D., et al., J. MoI. Biol. 222 (1991) 581-597). The techniques of Cole et al. and Boerner et al. are also available for the preparation of human monoclonal antibodies (Cole et al., Monoclonal Antibodies and Cancer Therapy, Alan R. Liss, p. 77 (1985); and Boerner, P., et al., J. Immunol. 147 (1991) 86-95). As already mentioned for chimeric and humanized antibodies according to the invention the term "human antibody" as used herein also comprises such antibodies which are modified in the constant region to generate the properties according to the invention, especially in regard to C1q binding and/or FcR binding, e.g. by "class switching" i.e. change or mutation of Fc parts (e.g. from IgG1 to IgG4 and/or IgG1/IgG4 mutation).

The term "recombinant human antibody", as used herein, is intended to include all human antibodies that are prepared, expressed, created or isolated by recombinant means, such as antibodies isolated from a host cell such as a NSO or CHO cell or from an animal (e.g. a mouse) that is transgenic for human immunoglobulin genes or antibodies expressed using a recombinant expression vector transfected into a host cell. Such recombinant human antibodies have variable and constant regions in a rearranged form. The recombinant human antibodies according to the invention have been subjected to in vivo somatic hypermutation. Thus, the amino acid sequences of the VH and VL regions of the recombinant antibodies are sequences that, while derived from and related to human germ line VH and VL sequences, may not naturally exist within the human antibody germ line repertoire in vivo.

The "variable domain" (variable domain of a light chain (VL), variable region of a heavy chain (VH)) as used herein denotes each of the pair of light and heavy chains which is involved directly in binding the antibody to the target. The domains of variable human light and heavy chains have the same general structure and each domain comprises four framework (FR) regions whose sequences are widely conserved, connected by three "hypervariable regions" (or complementarity determining regions, CDRs). The framework regions adopt a β-sheet conformation and the CDRs may form loops connecting the β-sheet structure. The CDRs in each chain are held in their three-dimensional structure by the framework regions and form together with the CDRs from the other chain the target binding site. The antibody heavy and light chain CDR3 regions play a particularly important role in the binding specificity/affinity of the antibodies according to the invention and therefore provide a further object of the invention.

The terms "hypervariable region" or "target-binding region of an antibody" when used herein refer to the amino acid residues of an antibody which are responsible for target-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. CDRs on each chain are separated by such framework amino acids. Especially, CDR3 of the heavy chain is the region which contributes most to target binding. CDR and FR regions are determined according to the standard definition of Kabat et al., Sequences of Proteins of Immunological Interest, 5th ed., Public Health Service, National Institutes of Health, Bethesda, MD (1991).

The term "epitope" includes any polypeptide determinant capable of specific binding to an antibody. In certain embodiments, epitope determinant include chemically active surface groupings of molecules such as amino acids, sugar side chains, phosphoryl, or sulfonyl, and, in certain embodiments, may have specific three dimensional structural characteristics, and or specific charge characteristics. An epitope is a region of a target that is bound by an antibody.

For the preparation of a bispecific antibody according to the invention there could be used separate vectors encoding each light and heavy chain or another appropriate amount of vectors. Such vectors can be used in transforming the host cell.

The term "nucleic acid or nucleic acid molecule", as used herein, is intended to include DNA molecules and RNA molecules. A nucleic acid molecule may be single-stranded or double-stranded, but preferably is double-stranded DNA.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included. Where distinct designations are intended, it will be clear from the context.

The term "transformation" as used herein refers to process of transfer of a vectors/nucleic acid into a host cell. If cells without formidable cell wall barriers are used as host cells, transfection is carried out e.g. by the calcium phosphate precipitation method as described by Graham and Van der Eh, Virology 52 (1978) 546ff. However, other methods for introducing DNA into cells such as by nuclear injection or by protoplast fusion may also be used. If prokaryotic cells or cells which contain substantial cell wall constructions are used, e.g. one method of transfection is calcium treatment using calcium chloride as described by Cohen SN, et al, PNAS 1972, 69 (8): 2110-2114.

Recombinant production of antibodies using transformation is well-known in the state of the art and described, for example, in the review articles of Makrides, S. C, Protein Expr. Purif. 17 (1999) 183-202; Geisse, S., et al., Protein Expr. Purif. 8 (1996) 271-282; Kaufman, RJ., MoI. Biotechnol. 16 (2000) 151-161; Werner, R.G., et al., Arzneimittelforschung 48 (1998) 870-880 as well as in US6331415 and US4816567.

As used herein, "expression" refers to the process by which a nucleic acid is transcribed into mRNA and/or to the process by which the transcribed mRNA (also referred to as transcript) is subsequently being translated into peptides, polypeptides, or proteins. The transcripts and the encoded polypeptides are collectively referred to as gene product. If the polynucleotide is derived from genomic DNA, expression in a eukaryotic cell may include splicing of the mRNA.

A "vector" is a nucleic acid molecule, in particular self-replicating, which transfers an inserted nucleic acid molecule into and/or between host cells. The term includes vectors that function primarily for insertion of DNA or RNA into a cell (e.g., chromosomal integration), replication of vectors that function primarily for the replication of DNA or RNA, and expression vectors that function for transcription and/or translation of the DNA or RNA. Also included are vectors that provide more than one of the functions as described.

An "expression vector" is a polynucleotide which, when introduced into an appropriate host cell, can be transcribed and translated into a polypeptide. An "expression system" usually refers to a suitable host cell comprised of an expression vector that can function to yield a desired expression product.

The bispecific antibodies according to the invention are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression, nucleic acids encoding light and heavy chains or fragments thereof are inserted into expression vectors by standard methods. Expression is performed in appropriate prokaryotic or eukaryotic host cells like CHO cells, NSO cells, SP2/0 cells, HEK293 cells, COS cells, yeast, or E.coli cells, and the antibody is recovered from the cells (supernatant or cells after lysis).The bispecific antibodies may be present in whole cells, in a cell lysate, or in a partially purified or substantially pure form. Purification is performed in order to eliminate other cellular components or other contaminants, e.g. other cellular nucleic acids or proteins, by standard techniques, including alkaline/SDS treatment, column chromatography and others well known in the art. See Ausubel, F., et al., ed., Current Protocols in Molecular Biology, Greene Publishing and Wiley Interscience, New York (1987).

Expression in NS0 cells is described by, e.g., Barnes, L.M., et al., Cytotechnology 32 (2000) 109-123; and Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; and Norderhaug, L., et al., J. Immunol. Methods 204 (1997) 77-87. A preferred transient expression system (HEK293) is described by Schlaeger, E.-J., and Christensen, K., in Cytotechnology 30 (1999) 71-83 and by Schlaeger, E.-J., in J. Immunol. Methods 194 (1996) 191-199.

The control sequences that are suitable for prokaryotes, for example, include a promoter, optionally an operator sequence, and a ribosome binding site. Eukaryotic cells are known to utilize promoters, enhancers and polyadenylation signals.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the

DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The bispecific antibodies are suitably separated from the culture medium by conventional immunoglobulin purification procedures such as, for example, protein A-Sepharose, hydroxylapatite chromatography, gel electrophoresis, dialysis, or affinity chromatography. DNA or RNA encoding the monoclonal antibodies is readily isolated and sequenced using conventional procedures. The hybridoma cells can serve as a source of such DNA and RNA. Once isolated, the DNA may be inserted into expression vectors, which are then transfected into host cells such as HEK293 cells, CHO cells, or myeloma cells that do not otherwise produce immunoglobulin protein, to obtain the synthesis of recombinant monoclonal antibodies in the host cells.

T cell bispecific (TCB) binders have very high concentration/tumor-cell-receptor-occupancy dependent potency in cell killing (e.g. EC50 in in vitro cell killing assays in the sub- or low picomolar range; Dreier et al. Int J Cancer 2002), T-cell bispecific binder (TCB) are given at much lower doses than conventional monospecific antibodies. For example, blinatumomab (CD19xCD3) is given at a continuous intravenous dose of 5 to 15 µg/m²/day (i.e. only 0.035 to 0.105 mg/m²/week) for treatment of acute lymphocytic leukemia or 60 µg/m²/day for treatment of Non Hodgkin Lymphoma, and the serum concentrations at these doses are in the range of 0.5 to 4 ng/ml (Klinger et al., Blood 2012; Topp et al., J Clin Oncol 2011; Goebeler et al. Ann Oncol 2011). Because low doses of TCB can exert high efficacy in patients, it is envisaged that for an antibody according to the invention subcutaneous administration is possible and preferred in the clinical settings (preferably in the dose range of 0.25 to 2.5 mg/m²/week). Even at these low concentrations/doses/receptor occupancies, TCB can cause considerable adverse events (Klinger et al., Blood 2012). Therefore it is critical to control tumor cell occupancy/coverage. Another advantage of the antibody according to the invention is based on the inclusion of an Fc portion, which increases the elimination half-life up to ∼12 days or even more and offers the opportunity of once or twice/week administrations as compared to TCBs without an Fc portion (e.g. blinatumomab) which are required to be given intravenously and continuously with a pump carried by patients.

With the CD19xCD3 T-cell bispecific (TCB) antibody blinatumomab response rates up to 80% have been shown in patients with relapsed/refractory Acute Lymphocytic Leukemia ALL, As for ALL for Multiple Myeloma and other plasma cell diseases there is still a high medical need. Despite all today available treatment, five years after first diagnosis approx 60% of Multiple Myeloma patients already died. There is still a need for an effective treatment for patients with Multiple Myeloma.

The antibodies according to the invention have unique features and advantages to e.g. blinatumomab and to published BCMAxCD3 TCB antibodies:
- long elimination half-life (days instead of hours)
- convenient twice or once a week administration (instead of administration by a pump to be carried for weeks/months by the patient)
- molecular format and structure providing high stability and low aggregation of the BCMAxCD3 TCB antibodies
- optimization of the molecular structures to enable high quality manufacturing and to facilitate purification by the use of a common light chain according to the invention and preferably knob into hole technology to improve correct heavy chain pairing, e.g. preferably Pro 329 and L234A and L235A amino acid substitutions in CH3 of Fc to avoid potential side effects from interaction with complement system and/or with FcR carrying effector cells.

In the following specific embodiments of the invention are listed:
1. An antibody light chain, comprising as CDRs CDR1L of SEQ ID NO:6, CDR2L of SEQ ID NO:7, and CDR3L of SEQ ID NO:8, for use as common light chain in a bispecific antibody specifically binding to the extracellular domain of human BCMA (further named also as "BCMA") and human CD3 (further named also as "CD3"), wherein the heavy chain of the CD3 binding part comprises as CDRs CDR1H of SEQ ID NO:3, CDR2H of SEQ ID NO:4, and CDR3H of SEQ ID NO:5.
2. The antibody light chain according to embodiment 1, comprising as variable light chain domain VL a VL of SEQ ID NO:2.
3. The antibody light chain according to embodiment 1 or 2, comprising as variable light chain of SEQ ID NO:29 for use as common light chain in a bispecific antibody specifically binding to the extracellular domain of human BCMA and human CD3, wherein the heavy chain of the CD3 binding part comprises as CDRs CDR1H of SEQ ID NO:3, CDR2H of SEQ ID NO:4, and CDR3H of SEQ ID NO:5.
4. A bispecific antibody specifically binding to the extracellular domain of human and human CD3, characterized in that the heavy chain of the CD3 binding part comprises as CDRs CDR1H of SEQ ID NO:3, CDR2H of SEQ ID NO:4, and CDR3H of SEQ ID NO:5 and an antibody light chain, comprising as CDRs CDR1L of SEQ ID NO:6, CDR2L of SEQ ID NO:7, and CDR3L of SEQ ID NO:8 is the common light chain.
5. The antibody according to embodiment 4, characterized in that the heavy chain of the CD3 binding part comprises as CDRs CDR1H of SEQ ID NO:3, CDR2H of SEQ ID NO:4, and CDR3H of SEQ ID NO:5 and an antibody light chain comprising as variable light chain domain VL a VL of SEQ ID NO:2 is the common light chain.
6. The antibody according to embodiment 4 or 5, characterized in that the heavy chain of the CD3 binding part comprises as CDRs CDR1H of SEQ ID NO:3, CDR2H of SEQ ID NO:4, and CDR3H of SEQ ID NO:5 and an antibody light chain of SEQ ID NO:29 is the common light chain.
7. The antibody according to any one of embodiments 4 to 6, characterized in comprising as variable heavy chain domain VH of the CD3 binding part a VH of SEQ ID NO: 1.
8. The antibody according to any one of embodiments 4 to 7, characterized in comprising as CDRs of the BCMA binding part a CDR set selected from the group consisting of:
   a) CDR1H of SEQ ID NO:18, CDR2H of SEQ ID NO:21, and CDR3H of SEQ ID NO:24 (set pSCHLI333)
   b) CDR1H of SEQ ID NO:19, CDR2H of SEQ ID NO:22, and CDR3H of SEQ ID NO:25 (set pSCHLI372)
   c) CDR1H of SEQ ID NO:20, CDR2H of SEQ ID NO:23, and CDR3H of SEQ ID NO:26 (set pSCHLI373)
   d) CDR1H of SEQ ID NO:35, CDR2H of SEQ ID NO:36, and CDR3H of SEQ ID NO:37 (set 83A10)
9. The antibody according to any one of embodiments 4 to 7, characterized in comprising as variable heavy chain domain VH of the BCMA binding part a VH of SEQ ID NO: 15, 16, 17 or 34 (83A10)
10. The antibody according to any one of embodiments 4 to 9, characterized in that the affinity to human BCMA of said bispecific antibody is 200 nM or lower, measured at an antibody concentration of 25 nM in presence of human BCMA Fc fusion at a concentration of 500 nM or lower in an affinity setup surface plasmon resonance assay.
11. The antibody according to any one of embodiments 4 to 10, characterized in that the potency (EC50) to kill BCMA-positive H929 cells (ATCC CRL-9068) of said bispecific antibody is measured as 2 nM or lower, when used at concentrations of 100 nM or lower, in presence of human PBCMs and H929 cells at a E:T ratio of 10:1 for 24h, in redirected T-cell killing LDH release assay.
12. The antibody according to any one of embodiments 4 to 11, characterized in that the affinity to human BCMA of said BCMA binding part, measured in an antibody of human IgG1 type, wherein the light chain is of SEQ ID NO:29, is 200 nM or lower at an antibody concentration of 25 nM in presence of human BCMA Fc fusion at a concentration 500 nM or lower in an affinity setup surface plasmon resonance assay.
13. A bispecific antibody according to any one of embodiments 4 to 12, characterized in consisting of one Fab fragment of an antibody specifically binding to CD3, and one Fab fragment of an antibody specifically binding to BCMA and a Fc part, wherein said CD3-Fab and said BCMA-Fab are linked via their C-termini to the hinge region of said Fc part and which said CD3-Fab and said BCMA-Fab comprise said common light chains for use according to the invention (Figure 1A).
14. The antibody according to any one of embodiments 4 to 12, characterized in consisting of two BCMA-Fabs, one CD3 Fab and a Fc part, wherein the first BCMA-Fab and the CD3-Fab are linked via their C-termini to the hinge region of said Fc part and said second BCMA-Fab is linked with its C-terminus to the N-terminus of said first BCMA-Fab. The CD3-Fab and BCMA-Fabs comprise said common light chains for use according to the invention (Figure 1B).
15. The antibody according to any one of embodiments 4 to 12, characterized in consisting of two BCMA-Fabs and a Fc part, wherein said BCMA-Fabs are linked via their C-termini to the hinge region of said Fc part and said CD3-Fab is linked with its C-terminus to the N-terminus of one BCMA-Fab. Said CD3-Fab and BCMA-Fabs comprise said common light chains for use according to the invention (Figure 1C).
16. The antibody according to any one of embodiments 4 to 12, characterized in consisting of one CD3-Fab and one BCMA Fab, which is linked via its C-terminus to the hinge region of said Fc part and a BCMA-Fab, which is linked with its C-terminus to the N-terminus of said CD3-Fab. Said CD3-Fab and BCMA-Fab comprise said common light chains for use according to the invention (Figure 1D).
17. The antibody according to any one of embodiments 4 to 12, characterized in consisting of one BCMA-Fab and one CD3 Fab, wherein said BCMA Fab is linked via its C-terminus to the hinge region of said Fc part and said CD3-Fabis linked with its C-terminus to the N-terminus of said BCMA-Fab. Said CD3-Fab and BCMA-Fab comprise said common light chains for use according to the invention (Figures IE).
18. The antibody according to any one of embodiments 4 to 12, characterized in consisting of two BCMA-Fabs, wherein the first BCMA-Fab is linked via its C-terminus to the N-terminus of the second BCMA-Fab, and wherein the second BCMA-Fab is linked via its C-terminus to the N-terminus of the CD3-Fab. The CD3-Fab and both BCMA-Fabs comprise said common light chains for use according to the invention (Figures IF).
19. The antibody according to any one of embodiments 4 to 12, characterized in consisting of one BCMA-Fab and one CD3 Fab, wherein the BCMA Fab which is linked with its C-terminus to the N-terminus of the CD3-Fab. The CD3-Fab and BCMA-Fab comprise said common light chains for use according to the invention (Figures 1G).
20. An antibody according to any one of embodiments 4 to 19, characterized in that the CH3 domain of one heavy chain and the CH3 domain of the other heavy chain each meet at an interface which comprises an original interface between the antibody CH3 domains; wherein said interface is altered to promote the formation of the bispecific antibody, wherein the alteration is characterized in that:
   a) the CH3 domain of one heavy chain is altered, so that within the original interface the CH3 domain of one heavy chain that meets the original interface of the CH3 domain of the other heavy chain within the bispecific antibody, an amino acid residue is replaced with an amino acid residue having a larger side chain volume, thereby generating a protuberance within the interface of the CH3 domain of one heavy chain which is positionable in a cavity within the interface of the CH3 domain of the other heavy chain and
   b) the CH3 domain of the other heavy chain is altered, so that within the original interface of the second CH3 domain that meets the original interface of the first CH3 domain within the bispecific antibody an amino acid residue is replaced with an amino acid residue having a smaller side chain volume, thereby generating a cavity within the interface of the second CH3 domain within which a protuberance within the interface of the first CH3 domain is positionable.
21. A method for the preparation of an a bispecific antibody according to any one of embodiments 4 to 20 comprising the steps of
   a) transforming a host cell with vectors comprising nucleic acid molecules encoding the light chain and heavy chain of an antibody according to any one of embodiments 4 to 20,
   b) culturing the host cell under conditions that allow synthesis of said antibody molecule; and
   c) recovering said antibody molecule from said culture.
22. A host cell comprising vectors comprising nucleic acid molecules encoding the light chain and heavy chains of an antibody according to any one of embodiments 4 to 20.
23. A pharmaceutical composition comprising the antibody according to any one of embodiments 4 to 20 and a pharmaceutically acceptable excipient.
24. The antibody according to any one of embodiments 4 to 20 or the pharmaceutical composition of embodiment 23 for use as a medicament.
25. The antibody according to any one of embodiments 4 to 20 or the pharmaceutical composition of embodiment 23 for use as a medicament in the treatment of plasma cell disorders like Multiple Myeloma.
26. The antibody according to any one of embodiments 4 to 20 or the pharmaceutical composition of embodiment 28 for use as a medicament in the treatment of multiple myeloma.
27. The antibody according to any one of embodiments 4 to 20 or the pharmaceutical composition of embodiment 28 for the treatment of plasma cell disorders like multiple myeloma or other B-cell disorders expressing BCMA.
28. The antibody according to any one of embodiments 4 to 20, characterized in comprising in the human IgG1 Fc part amino acid substitution of Pro329 with glycine or arginine and/or substitutions L234A and L235A.
29. The antibody light chain for use as common light chain in a bispecific antibody according to embodiment 1, wherein said bispecific antibody is an antibody according to embodiments 4 to 20.
30. Use of an antibody light chain, comprising as CDRs CDR1L of SEQ ID NO:6, CDR2L of SEQ ID NO:7, and CDR3L of SEQ ID NO:8, as common light chain in a bispecific antibody specifically binding to the extracellular domain of human BCMA (further named also as "BCMA") and human CD3 (further named also as "CD3"), wherein the heavy chain of the CD3 binding part comprises as CDRs CDR1H of SEQ ID NO:3, CDR2H of SEQ ID NO:4, and CDR3H of SEQ ID NO:5.
31. The use according to embodiment 30, characterized in that the light chain comprises as variable light chain domain VL a VL of SEQ ID NO:2.
32. The use according to embodiment 30 or 31, characterized in that the light chain comprises as light chain the light chain of SEQ ID NO:29.

The following examples, sequence listing and figures are provided to aid the understanding of the present invention, the true scope of which is set forth in the appended claims. It is understood that modifications can be made in the procedures set forth without departing from the spirit of the invention.

### Materials & general methods

### Recombinant DNA techniques

Standard methods were used to manipulate DNA as described in Sambrook, J. et al., Molecular cloning: A laboratory manual; Cold Spring Harbor Laboratory Press, Cold Spring Harbor, New York, 1989. The molecular biological reagents were used according to the manufacturer's instructions. General information regarding the nucleotide sequences of human immunoglobulins light and heavy chains is given in: Kabat, E.A. et al., (1991) Sequences of Proteins of Immunological Interest, 5th ed., NIH Publication No. 91-3242. Amino acids of antibody chains were numbered and referred to according to Kabat, E.A., et al., Sequences of Proteins of Immunological Interest, 5th ed., Public Health Service, National Institutes of Health, Bethesda, MD, (1991).

### Gene synthesis

a) Desired gene segments were prepared from oligonucleotides made by chemical synthesis. The 600 - 1800 bp long gene segments, which were flanked by singular restriction endonuclease cleavage sites, were assembled by annealing and ligation of oligonucleotides including PCR amplification and subsequently cloned via the indicated restriction sites e.g. Kpnl/ Sad or Ascl/Pacl into a pPCRScript (Stratagene) based pGA4 cloning vector. The DNA sequences of the subcloned gene fragments were confirmed by DNA sequencing. Gene synthesis fragments were ordered according to given specifications at Geneart/Life Technologies (Regensburg, Germany).
b) Desired gene segments where required were either generated by PCR using appropriate templates or were synthesized by Geneart AG (Regensburg, Germany) from synthetic oligonucleotides and PCR products by automated gene synthesis. The gene segments flanked by singular restriction endonuclease cleavage sites were cloned into standard expression vectors or into sequencing vectors for further analysis. The plasmid DNA was purified from transformed bacteria using commercially available plasmid purification kits. Plasmid concentration was determined by UV spectroscopy. The DNA sequence of the subcloned gene fragments was confirmed by DNA sequencing. Gene segments were designed with suitable restriction sites to allow sub-cloning into the respective expression vectors. If required, protein coding genes were designed with a 5'-end DNA sequence coding for a leader peptide which targets proteins for secretion in eukaryotic cells.

### DNA sequence determination

DNA sequences were determined by double strand sequencing.

### DNA and protein sequence analysis and sequence data management

The Clone Manager (Scientific & Educational Software) software package version 9.2 was used for sequence mapping, analysis, annotation and illustration.

### Expression vectors

a) For the generation of anti-BCMA antibody expression vectors, the variable regions of heavy and light chain DNA sequences were subcloned in frame with either the human IgG1 constant heavy chain or the hum IgG1 constant light chain pre-inserted into the respective generic recipient expression vector optimized for expression in mammalian cell lines. The antibody expression is driven by a chimeric MPSV promoter comprising a CMV enhancer and a MPSV promoter followed by a 5' UTR, an intron and a Ig kappa MAR element. The transcription is terminated by a synthetic polyA signal sequence at the 3' end of the CDS. All vectors carry a 5'-end DNA sequence coding for a leader peptide which targets proteins for secretion in eukaryotic cells. In addition each vector contains an EBV OriP sequence for episomal plasmid replication in EBV EBNA expressing cells.
b) For the generation of BCMAxCD3 bispecific antibody vectors, the IgG1 derived bispecific molecules consist at least of two antigen binding moieties capable of binding specifically to two distinct antigenic determinants CD3 and BCMA. The antigen binding moieties are Fab fragments composed of a heavy and a light chain, each comprising a variable and a constant region. The light chain of Fab CD3 and Fab BCMA are identical to avoid LC mispairing. The bispecific molecule design was monovalent for CD3 and bivalent for BCMA where one Fab fragment was fused to the N-terminus of the inner Fab (2+1). The bispecific molecule contained an Fc part in order for the molecule to have a long half-life. A schematic representation of the constructs is given in Figure 1; the preferred sequences of the constructs are shown in SEQ ID NOs 29 to 31. The molecules were produced a polymer-based cotransfection of HEK293 EBNA cells growing in suspension with the mammalian expression vectors. For preparation of 2+1 Fab-IgG constructs, cells were transfected with the corresponding expression vectors in a 1:1:2:1 ratio (heavy chain: modified heavy chain: light chain: modified light chain = 1:1:2:1; Ratio standard antibody: heavy chain : light chain = 1:1).

### Cell culture techniques

Standard cell culture techniques are used as described in Current Protocols in Cell Biology (2000), Bonifacino, J. S., Dasso, M., Harford, J.B., Lippincott-Schwartz, J. and Yamada, K.M. (eds.), John Wiley & Sons, Inc.

### Transient expression in HEK293 cells (HEK293-EBNA system)

Bispecific antibodies were expressed by transient polymer-based cotransfection of the respective mammalian expression vectors in HEK293-EBNA cells, which were cultivated in suspension. One day prior to transfection the HEK293-EBNA cells were seeded at 1.5 Mio viable cells/mL in Ex-Cell medium, supplemented with 6 mM of L-Glutamine. For every mL of final production volume 2.0 Mio viable cells were centrifuged (5 minutes at 210 x g). The supernatant was aspirated and the cells resuspended in 100 µL of CD CHO medium. The DNA for every mL of final production volume was prepared by mixing 1 µg of DNA (Ratio Bispecific Antibody Production: heavy chain: modified heavy chain: light chain: modified light chain = 1:1:2:1; Ratio standard antibody: heavy chain : light chain = 1:1) in 100 µL of CD CHO medium. After addition of 0.27 µL of PEI solution (1 mg/mL) the mixture was vortexed for 15 seconds and left at room temperature for 10 minutes. After 10 minutes, the resuspended cells and DNA/PEI mixture were put together and then transferred into an appropriate container which was placed in a shaking device (37°C, 5% CO₂). After a 3 hours incubation time 800 µL of Ex-Cell Medium, supplemented with 6 mM L-Glutamine, 1.25 mM valproic acid and 12.5% Pepsoy (50 g/L), was added for every mL of final Production volume. After 24 hours, 70 µL of feed solution was added for every mL of final production volume. After 7 days or when the cell viability was equal or lower than 70%, the cells were separated from the supernatant by centrifugation and sterile filtration.

### Protein determination

Determination of the antibody concentration was done by measurement of the absorbance at 280 nm, using the theoretical value of the absorbance of a 0.1% solution of the antibody. This value was based on the amino acid sequence and calculated by GPMAW software (Lighthouse data).

### SDS-PAGE

The NuPAGE® Pre-Cast gel system (Invitrogen) is used according to the manufacturer's instruction. In particular, 10% or 4-12% NuPAGE® Novex® Bis-TRIS Pre-Cast gels (pH 6.4) and a NuPAGE® MES (reduced gels, with NuPAGE® Antioxidant running buffer additive) or MOPS (non-reduced gels) running buffer is used.

### Protein purification

The antibodies were purified by an affinity step and one polishing step, being size exclusion chromatography.

### By protein A affinity chromatography

For the affinity step the supernatant was loaded on a protein A column (HiTrap Protein A FF , 5 mL, GE Healthcare) equilibrated with 6 CV 20 mM sodium phosphate, 20 mM sodium citrate, pH 7.5. After a washing step with the same buffer the antibody was eluted from the column by step elution with 20 mM sodium phosphate, 100 mM sodium chloride, 100 mM Glycine, pH 3.0. The fractions with the desired antibody were immediately neutralized by 0.5 M Sodium Phosphate, pH 8.0 (1:10), pooled and concentrated by centrifugation. The concentrate was sterile filtered and processed further by size exclusion chromatography.

### By size exclusion chromatography

For the size exclusion step the concentrated protein was injected on a XK16/60 HiLoad Superdex 200 column (GE Healthcare), and 20 mM Histidine, 140 mM Sodium Chloride, pH 6.0 with or without Tween20 as formulation buffer. The fractions containing the monomers were pooled, concentrated by centrifugation and sterile filtered into a sterile vial.

### Measurement of purity and monomer content

Purity and monomer content of the final protein preparation was determined by CE-SDS (Caliper LabChip GXII system (Caliper Life Sciences)) resp. HPLC (TSKgel G3000 SW XL analytical size exclusion column (Tosoh)) in a 25 mM potassium phosphate, 125 mM Sodium chloride, 200 mM L-arginine monohydrochloride, 0.02 % (w/v) Sodium azide, pH 6.7 buffer.

### Isolation of primary human pan T cells from PBMCs

Peripheral blood mononuclear cells (PBMCs) were prepared by Histopaque density centrifugation from enriched lymphocyte preparations (buffy coats) obtained from local blood banks or from fresh blood from healthy human donors. Briefly, blood was diluted with sterile PBS and carefully layered over a Histopaque gradient (Sigma, H8889). After centrifugation for 30 minutes at 450 x g at room temperature (brake switched off), part of the plasma above the PBMC containing interphase was discarded. The PBMCs were transferred into new 50 ml Falcon tubes and tubes were filled up with PBS to a total volume of 50 ml. The mixture was centrifuged at room temperature for 10 minutes at 400 x g (brake switched on). The supernatant was discarded and the PBMC pellet washed twice with sterile PBS (centrifugation steps at 4°C for 10 minutes at 350 x g). The resulting PBMC population was counted automatically (ViCell) and stored in RPMI1640 medium, containing 10% FCS and 1% L-alanyl-L-glutamine (Biochrom, K0302) at 37°C, 5% CO₂ in the incubator until assay start.

T cell enrichment from PBMCs was performed using the Pan T Cell Isolation Kit II (Miltenyi Biotec #130-091-156), according to the manufacturer's instructions. Briefly, the cell pellets were diluted in 40 µḯ cold buffer per 10 million cells (PBS with 0.5% BSA, 2 mM EDTA, sterile filtered) and incubated with 10 µḯ Biotin- Antibody Cocktail per 10 million cells for 10 min at 4°C. 30 µḯ cold buffer and 20 µḯ Anti-Biotin magnetic beads per 10 million cells were added, and the mixture incubated for another 15 min at 4°C. Cells were washed by adding 10-20x the current volume and a subsequent centrifugation step at 300 x g for 10 min. Up to 100 million cells were resuspended in 500 µḯ buffer. Magnetic separation of unlabeled human pan T cells was performed using LS columns (Miltenyi Biotec #130-042-401) according to the manufacturer's instructions. The resulting T cell population was counted automatically (ViCell) and stored in AIM-V medium at 37°C, 5% C0₂ in the incubator until assay start (not longer than 24 h).

### Isolation of primary human naive T cells from PBMCs

Peripheral blood mononuclar cells (PBMCs) were prepared by Histopaque density centrifugation from enriched lymphocyte preparations (buffy coats) obtained from local blood banks or from fresh blood from healthy human donors. T-cell enrichment from PBMCs was performed using the Naive CD8⁺ T cell isolation Kit from Miltenyi Biotec (#130-093-244), according to the manufacturer's instructions, but skipping the last isolation step of CD8⁺ T cells (also see description for the isolation of primary human pan T cells).

### Examples

### Example 1: Generation of anti-BCMA antibodies

### Example 1.1: Production of antigens and tool reagents

### Example 1.1.1: Recombinant, soluble, human BCMA extracellular domain

The extracellular domains of human, cynomolgus and murine BCMA that were used as antigens for phage display selections were transiently expressed as N-terminal monomeric Fc-fusion in HEK EBNA cells and in vivo site-specifically biotinylated via co-expression of BirA biotin ligase at the avi-tag recognition sequence located at the C-terminus of the Fc portion carrying the receptor chain (Fc knob chain). The extracellular domains of human, cynomolgus and murine BCMA comprised methionine 4 to asparagine 53, methionine 4 to asparagine 52, and alanine 2 to threonine 49, respectively. These were N-terminally fused to the hinge of a human IgG1 enabling heterodimerization with an unfused human IgG1 Fc portion (hole chain) by knobs-into-holes technology.

For recovering of the extracellular domain of BCMA the following primers were used:
AAGCTTGGATCCATGTTGCAGATGGCTGGGCAGTGCTCC-3 (SEQ ID NO:9) incorporating a BamH1 site (bold, underlined) and
reverse primer 5-GAATTCGCGGCCGCTCATCCTTTCACTGAATTGGTCACACTTGCATTAC-3 (SEQ ID NO: 10)
primer 5-ACGTTAGATCTCCACTCAGTCCTGCATCTTGTTCCAGTTAAC-3 (SEQ ID NO:11) and reverse primer 5-AACGTTGCGGCCGCTAGTTTCACAAACCCCAGG-3 (SEQ ID NO:12)
GAATTCAAGCTTGCCACCATGTTGCAGATGGCTGGGCAGTGCTCC-3 (SEQ ID NO:13) including a HindIII restriction site (bold, underlined) and Kozak consensus sequence and
reverse primer 5-GAATTCTCTAGATTACCTAGCAGAAATTGATTTCTCTATCTCCGTAGC-3 (SEQ ID NO:14).
Gene synthesis can be also used to obtain the extracellular domain of BCMA.

### Example 1.2: BCMA-expressing cells as tools

### Example 1.2.1: Human myeloma cell line expressing BCMA on their surface

BCMA expression was assessed on four human myeloma cell lines (NCI-H929, RPMI-8226, U266B1 and L-363) by flow cytometry. NCI-H929 cells ((H929) ATCC® CRL-9068™) were cultured in 80-90% RPMI 1640 with 10-20% heat-inactivated FCS and could contain 2 mM L-glutamine, 1 mM sodium pyruvate and 50 µM mercaptoethanol. RPMI-8226 cells ((RPMI) ATCC® CCL-155™) were cultured in a media containing 90% RPMI 1640 and 10% heat-inactivated FCS. U266B1 ((U266) ATCC® TIB-196^{™}) cells were cultured in RPMI-1640 medium modified to contain 2 mM L-glutamine, 10 mM HEPES, 1 mM sodium pyruvate, 4500 mg/L glucose, and 1500 mg/L sodium bicarbonate and 15% heat-inactivated FCS. L-363 cell line (Leibniz Institute DSMZ - German collection of microorganisms and cell cultures; DSMZ No. ACC 49) was cultured in 85% RPMI 1640 and 15% heat-inactivated FCS. Briefly, cells were harvested, washed, counted for viability, resuspended at 50,000 cells/well of a 96-well round bottom plate and incubated with anti-human BCMA antibody (Abcam, #ab54834, mouse IgG1) at 10 µg/ml for 30 min at 4°C (to prevent internalization). A mouse IgG1 was used as isotype control (BD Biosciences, #554121). Cells were then centrifuged (5 min at 350 x g), washed twice and incubated with the FITC-conjugated anti mouse secondary antibody for 30 min at 4°C. At the end of incubation time, cells were centrifuged (5 min at 350 x g), washed twice with FACS buffer, resuspended in 100 ul FACS buffer and analyzed on a CantoII device running FACS Diva software. The relative quantification of BCMA receptor number on the surface membrane of H929, U266B1, RPMI-8226 and L-363 myeloma cell lines was assessed by QIFIKIT analysis (Dako, #K0078, following manufacturer's instructions). H929 cells expressed human BCMA with the highest density, up to 3.8 to 27-fold higher than other myeloma cell lines. H929 is considered as a BCMA^{hi}-expressing myeloma cell line as compared to U266 which is BCMA^{med/lo}-expressing myeloma cells and RPMI-8226 and L363 which are BCMA^{lo}-expressing myeloma cells. Table 3 summarizes the relative BCMA receptor number on the cell surface of human multiple myeloma cell lines.

**Table 3: Quantification of BCMA receptor number on cell surface of NCI-H929, U266B1, RPMI-8226 and L-363 human myeloma cell lines**

| **Myeloma cell lines** | **Relative binding sites per cell** |
|---|---|
| H929 | 50000 |
| U266 | 13000 |
| RPMI-8226 | 2000 |
| L363 | 1800 |

### Example 1.3: Obtaining BCMA binders out of an in vitro, recombinant library

### Example 1.3.1: Construction of common light chain Fab-Libraries

Antibody libraries in the Fab-format are constructed on the basis of the humanized anti-CD3 antibody light chain (SEQ ID NO:29) were generated. Diversity was only introduced in the heavy chain. Six different heavy chain frameworks were used: VH1-46, VH1-69, VH3-15, VH3-23, VH4-59 and VH5-1. CDRs 1, 2 and 3 were randomized and each heavy chain library was combined with the non-randomized light chain. Libraries were generated as described by Nissim et al EMBO J. 1994 Feb 1;13(3):692-8, and Silacci et al. Proteomics. 2005 Jun;5(9):2340-50.

### Example 1.3.2: Selection of anti-BCMA Fab clones

Anti-BCMA Fabs were established by phage display from synthetic Fab libraries consisting one constant VL and six different human VH sequences

**Table 4: Anti-BCMA clones and respective VL / VH pairings**

| **Fab clone** | **VL domain** | **VH domain** |
|---|---|---|
| pSCHLI372 | QAVVTQEPSLTVSPGGTVTLTCG SSTGAVTTSNYANWVQEKPGQA FRGLIGGTNKRAPGTPARFSGSL LGGKAALTLSGAQPEDEAEYYC ALWYSNLWVFGGGTKLTVL (SEQ ID NO:8) | EVQLVESGGGLVKPGGSLRLSCA ASGFTFSNSGMIWVRQAPGKGLE WVGHIRSKTDGGTTDYAAPVKGR FTISRDDSKNTLYLQMNSLKTEDT AVYYCTTGGSGSFDYWGQGTLVT VSS (SEQ ID NO:16) |
| pSCHLI373 | QAVVTQEPSLTVSPGGTVTLTCG SSTGAVTTSNYANWVQEKPGQA FRGLIGGTNKRAPGTPARFSGSL LGGKAALTLSGAQPEDEAEYYC ALWYSNLWVFGGGTKLTVL (SEQ ID NO:8) | EVQLVESGGGLVKPGGSLRLSCA ASGFSFSNSWMNWVRQAPGKGL EWVGTIRQKTYGGTTDYAAPVKG RFTISRDDSKNTLYLQMNSLKTED TAVYYCTTGGLFGYWDYWGQGT LVTVSS (SEQ ID NO:17) |

Selection rounds (biopanning) were performed in solution according to the following pattern: 1) pre-clearing of ∼ 10¹² phagemid particles per library pool in immunotubes coated with 10ug/ml of an unrelated human IgG to deplete the libraries of antibodies recognizing the Fc-portion of the antigens; 2) incubation of the non-Fc-binding phagemid particles with 100nM biotinylated BCMA for 0.5 h in the presence of 100nM unrelated non-biotinylated Fc knobs-into-holes construct for further depletion of Fc-binders in a total volume of 2ml; 3) capture of biotinylated BCMA and specifically binding phage by splitting up and transferring the panning reaction into 16 wells on a neutravidin or streptavidin pre-coated microtiter plate for 20 min on a shaker; 4) washing of respective wells 10-30x with PBS/Tween20 and 10-30x with PBS using a plate washer; 5) optional competitive washing step by addition of 230nM murine APRIL to displace Fab clones that recognize the binding site of the natural ligand thus selecting for APRIL-non-competing phage antibodies; 6) elution of phage particles by addition of 125ul 100mM TEA (triethylamine) per well for 5-10 min and neutralization by addition of an equal volume of 1M Tris/HCl pH 7.4; 7) re-infection of log-phase E. coli TG1 cells with the eluted phage particles, infection with helperphage VCSM13, incubation on a shaker at 30°C overnight and subsequent PEG/NaCl precipitation of phagemid particles to be used in the next selection round.

Selections were carried out over 3 to 5 rounds using constant antigen concentrations of 100nM. Apart from selection campaigns during which only human BCMA was used as antigen, additional selection campaigns were carried out during which also cynomolgus or murine BCMA were used in an alternating fashion with human BCMA in order to select for cross-reactive antibodies. Moreover, as an alternative to streptavidin plate-based capture, capture of antigen : phage complexes was performed by addition of 5.4 × 10⁷ streptavidin-coated magnetic beads to the panning reaction followed by washing steps using respective magnets under the conditions described above.

Specific binders were identified by surface plasmon resonance-screening of Fab-containing bacterial culture supernatants using BioRad's ProteOn XPR36 biosensor. In brief, after infection of log-phase E. coli TG1 cells with the eluted phage particles, single colony forming units (cfu) were plated and picked for inoculation of 1ml expression cultures in 96-deep well plates. Fabs were captured from the supernatants on a ProteOn GLM chip that was derivatized with 8.000 - 10.000 RU of a goat anti-human IgG, F(ab')2 fragment specific polyclonal antibody (Jackson ImmunoResearch, # 109-005-006) in vertical orientation. Subsequently, human, cynomolgus and murine BCMA as well as an unrelated Fc knobs-into-holes construct were injected as analytes in horizontal orientation. Clones that exhibited significant binding responses to BCMA and did not bind the Fc-portion of the antigens, were bacterially expressed in a 0.5 liter culture volume, affinity purified and kinetically characterized by SPR-analysis using a one-shot-kinetics protocol on BioRad's ProteOn XPR36 biosensor.

### Example 2: BCMA binding assays: surface plasmon resonance

Assessment of binding of anti-BCMA antibodies to recombinant BCMA by surface plasmon resonance (SPR) as follow. All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany). The avidity of the interaction between anti-BCMA antibodies and recombinant BCMA Fc(kih) (human, cynomolgus and murine) was determined (Tables 5-7). Biotinylated recombinant human, cynomolgus and murine BCMA Fc(kih) were directly coupled on a SA chip following instructions (Biacore, Freiburg/Germany). The immobilization level ranged from 80 to 120 RU. The anti-BCMA antibodies were passed at a 4-fold concentration range (1.95 to 500 nM) with a flow of 30 µL/minutes through the flow cells over 120 seconds. The dissociation was monitored for 180 seconds. Bulk refractive index differences were corrected for by subtracting the response obtained on the reference flow cell. Here, the anti-BCMA antibodies were flown over an empty surface previously activated and deactivated as described in the standard amine coupling kit. Apparent kinetic constants were derived using the Biacore T200 Evaluation Software (v 1.0, Biacore AB, Uppsala/Sweden), to fit rate equations for 1:1 Langmuir binding by numerical integration, despite the bivalency of the interaction for comparison purposes.

**Table 5. Avidity values for comparison purposes only (Experiment 1)**

| **Analyte** | **Ligand** | **Kon[1/Ms]** | **Koff[1/s]** | **KD[nM]** |
|---|---|---|---|---|
| pSCHLI333 anti-BCMA IgG | huBCMA Fc(kih) | 4.55E+06 | 1.13E-02 | 2.5 |
| pSCHLI333 anti-BCMA IgG | cyBCMA Fc(kih) | 9.39E+06 | 1.24E-02 | 1.3 |
| pSCHLI333 anti-BCMA IgG | muBCMA Fc(kih) | 8.26E+06 | 1.62E-01 | 19.6 |

**Table 6. Avidity values for comparison purposes only (Experiment 2)**

| **Analyte** | **Ligand** | **Kon[1/Ms]** | **Koff[1/s]** | **KD[nM]** |
|---|---|---|---|---|
| pSCHLI372 anti-BCMA IgG | huBCMA Fc(kih) | 3.13E+05 | 2.66E-03 | 8.5 |
| pSCHLI372 anti-BCMA IgG | cyBCMA Fc(kih) | 7.85E+05 | 3.50E-03 | 4.5 |
| pSCHLI372 anti-BCMA IgG | muBCMA Fc(kih) | 1.30E+05 | 5.53E-01 | 42.7 |
| pSCHLI373 anti-BCMA IgG | huBCMA Fc(kih) | 1.40E+05 | 3.23E-03 | 23.0 |
| pSCHLI373 anti-BCMA IgG | cyBCMA Fc(kih) | 9.36E+04 | 9.28-04 | 9.9 |

**Table 7. Avidity values for comparison purposes only (Experiment 3)**

| **Analyte** | **Ligand** | **Kon[1/Ms]** | **Koff[1/s]** | **KD[nM]** |
|---|---|---|---|---|
| pSCHLI372 anti-BCMA IgG | huBCMA Fc(kih) | 8.03E+05 | 2.84E-03 | 3.6 |
| pSCHLI372 anti-BCMA IgG | cyBCMA Fc(kih) | 1.63E+06 | 4.69E-03 | 2.9 |
| pSCHLI372 anti-BCMA IgG | muBCMA Fc(kih) | 4.69E+05 | 1.21E-02 | 25.7 |
| pSCHLI373 anti-BCMA IgG | huBCMA Fc(kih) | 9.78E+04 | 1.18E-03 | 12.1 |
| pSCHLI373 anti-BCMA IgG | cyBCMA Fc(kih) | 9.39E+04 | 7.98E-04 | 8.5 |

The affinity of the interaction between anti-BCMA antibodies or BCMA-TCB CLC antibodies and recombinant human BCMA Fc(kih) was also determined. Anti-human Fab antibody (GE Healthcare) was directly coupled on a CM5 chip at pH 5.0 using the standard amine coupling kit (Biacore, Freiburg/Germany). The immobilization level was about 6500 RU. Anti-BCMA antibody or BCMA-TCB CLC antibody was captured for 90 seconds at 25 nM. Recombinant human BCMA Fc(kih) was passed at a 4-fold concentration range (1.95 to 500 nM) with a flow of 30 µL/minutes through the flow cells over 120 or 180 seconds. The dissociation was monitored for 120 or 400 seconds. Bulk refractive index differences were corrected for by subtracting the response obtained on reference flow cell. Here, recombinant BCMA was flown over a surface with immobilized anti-human Fab antibody but on which HBS-EP has been injected rather than anti-BCMA antibody or BCMA-TCB CLC antibody. Kinetic constants were derived using the Biacore T200 Evaluation Software (v 1.0, Biacore AB, Uppsala/Sweden), to fit rate equations for 1:1 Langmuir binding by numerical integration (Table 8).

**Table 8. Affinity constants determined by fitting rate equations for 1:1 Langmuir binding**

| **Ligand** | **Analyte** | **Kon[1/Ms]** | **Koff[1/s]** | **KD[nM]** |
|---|---|---|---|---|
| pSCHLI372 anti-BCMA IgG | huBCMA Fc(kih) | 4.59E+04 | 5.23E-03 | 114 |
| pSCHLI372 anti-BCMA IgG | cyBCMA Fc(kih) | 2.68E+04 | 7.52E-03 | 281 |
| pSCHLI372 anti-BCMA IgG | muBCMA Fc(kih) | 9.92E+04 | 1.30E-01 | 1310 |
| pSCHLI373 anti-BCMA IgG | huBCMA Fc(kih) | 4.22E+04 | 5.70E-03 | 135 |
| pSCHLI373 anti-BCMA IgG | cyBCMA Fc(kih) | 2.10E+04 | 1.12E-02 | 535 |
| pSCHLI373 anti-BCMA IgG | muBCMA Fc(kih) | 1.27E+05 | 9.19E-02 | 724 |
| pSCHLI333 BCMAxCD3 TCB | huBCMA Fc(kih) | 9.10E+03 | 1.49E-03 | 164 |
| pSCHLI333 BCMAxCD3 TCB | cyBCMA Fc(kih) | 1.44E+04 | 2.63E-03 | 183 |
| pSCHLI333 BCMAxCD3 TCB | muBCMA Fc(kih) | 4.83E+02 | 3.47E-03 | 7190 |
| pSCHLI372 BCMAxCD3 TCB | huBCMA Fc(kih) | 5.59E+04 | 3.52E-03 | 63 |
| pSCHLI372 BCMAxCD3 TCB | cyBCMA Fc(kih) | 4.58E+04 | 7.57E-03 | 165 |
| pSCHLI372 BCMAxCD3 TCB | muBCMA Fc(kih) | 1.42E+04 | 4.87E-03 | 344 |

### Example 3: Generation of bivalent anti-BCMA IgG antibody with the common light chain

To verify the hypothesis that the use of the common light chain could be applied to any BCMA antibodies, 83A10-CLC bivalent BCMA antibody was generated by substituting the native light chain of the bivalent BCMA antibody 83A10, previously described in WO/2014/122143, with the common light chain of SEQ ID NO:29. The anti-BCMA antibody comprising two heavy chains of 83A10 and two common light chains were produced using the general techniques for generation of bivalent IgG antibodies as described in the Material and general methods section. Affinity of 83A10-CLC IgG antibody to human BCMA was measured by SPR with methods similar to the ones described in Example 2. Table 9 depicts the binding of recombinant human BCMA Fc(kih) to 83A10-CLC BCMA IgG antibody.

**Table 9. Affinity constants determined by fitting rate equations for 1:1 Langmuir binding: Binding of recombinant BCMA Fc(kih) to 83A10-CLC anti-BCMA antibody**

| **Ligand** | **Analyte** | **Kon[1/Ms]** | **Koff[1/s]** | **KD[nM]** |
|---|---|---|---|---|
| 83A10 CLC anti-BCMA IgG | huBCMA Fc(kih) | 8.78E+04 | 2.13E-3 | 24.3 |

### Example 4: Specificity test of anti-BCMA IgG antibodies to huTACI-R and huBAFF-R

As members of the TNF-TNF-R superfamily, TACI and BAFF receptors are related to BCMA receptor with respectively 22% and 18.5% homology in the extracellular domain. Therefore, surface plasmon resonance (SPR) binding experiments were performed to examine the specificity of anti-BCMA IgG antibodies. All SPR experiments were performed on a Biacore T200 (GE Healthcare) at 25 °C with HBS-EP as running buffer (0.01 M HEPES pH 7.4, 0.15 M NaCl, 3 mM EDTA, 0.005% Surfactant P20). Fc fused huBCMA, huBAFF-R and huTACI-R were chemically immobilized with a high immobilization level (∼5000 RU) on different flow channels of a Biacore CM5 sensor chip at pH 5.0 using the standard amine coupling kit (GE Healthcare). Initially high concentrated solutions (3µM, dissolved in HBS-EP) of anti-BCMA IgG pSCHLI372 as well as a-huTACI-R IgG or a-huBAFF-R IgG as positive controls were injected (association time: 80s, dissociation time: 600s, flow: 30µl/min) to check if binding occurs. A positive binding event of the a-huTACI-R IgG to huTACI-R as well as for a-huBAFF-R IgG to huBAFF-R and anti-BCMA IgG antibodies to huBCMA indicated that all receptors were still recognized after immobilization. For anti-BCMA IgG antibodies binding with fast kinetic rate constants to huBAFF-R and/or huTACI-R, a careful examination of kinetic parameter with low immobilization levels (300 RU) was performed on a new CM5 sensor chip. Anti-BCMA IgG antibody dilutions at concentrations of 3000 - 93.75 nM (2-fold dilution in HBS-EP) are injected (association time: 80s, dissociation time: 300s, flow: 30µl/min), and sample(s) were tested in duplicate. Regeneration was also performed when applicable i.e no fast and complete dissociation. Kinetic evaluation of the interaction between anti-BCMA IgG antibodies and huBAFF-R or huTACI-R was performed by global fitting of the data to a 1:1 interaction model that includes a term for mass transport (Biacore T200 evaluation Version 1.0). A steady state analysis was also performed. As depicted in Figure 2, curve 1 corresponds to the signal on reference channel, curve 2 to the channel where the binding occurs (binding channel) and the curve 2-1 is the subtracted signal (binding channel - reference channel), meaning that this is the signal due to the binding event. As shown in Figure 2, the SPR binding assay clearly demonstrated that pSCHLI372 IgG did not bind to human TACI receptor. As positive control for binding, another BCMA IgG known to slightly bind to TACI receptor but with very fast on- and off-rate was used (data not shown).

### Example 5: Generation of anti-BCMA/anti-CD3 T cell bispecific antibodies

### Example 5.1: Anti-CD3 antibodies

The term "CD3ε or CD3" as used herein relates to human CD3ε described under UniProt P07766 (CD3E_HUMAN). The term "antibody against CD3, anti CD3 antibody" relates to an antibody binding to CD3ε. Preferably the antibody comprises a variable domain VH comprising the heavy chain CDRs of SEQ ID NO: 3, 4 and 5 as respectively heavy chain CDR1, CDR2 and CDR3 and a variable domain VL comprising the light chain CDRs of SEQ ID NO: 6, 7 and 8 as respectively light chain CDR1, CDR2 and CDR3. Preferably the antibody comprises the variable domains of SEQ ID NO:1 (VH) and SEQ ID NO:2 (VL).

Anti-CD3 antibody as described above was used to generate the T cell bispecific antibodies which were used in the following examples.

### Example 6: Production and purification of BCMA-TCB CLC Fc-containing (2+1) antibodies

For the production of the bispecific antibodies, bispecific antibodies were expressed by transient polymer-based cotransfection of the respective mammalian expression vectors in HEK293-EBNA cells, which were cultivated in suspension. One day prior to transfection the HEK293-EBNA cells were seeded at 1.5 Mio viable cells/mL in Ex-Cell medium, supplemented with 6 mM of L-Glutamine. For every mL of final production volume 2.0 Mio viable cells were centrifuged (5 minutes at 210 x g). The supernatant was aspirated and the cells resuspended in 100 µL of CD CHO medium. The DNA for every mL of final production volume was prepared by mixing 1 µg of DNA (Ratio Bispecific Antibody Production: heavy chain: modified heavy chain: light chain: modified light chain = 1:1:2:1; Ratio standard antibody: heavy chain : light chain = 1:1) in 100 µL of CD CHO medium. After addition of 0.27 µL of PEI solution (1 mg/mL) the mixture was vortexed for 15 seconds and left at room temperature for 10 minutes. After 10 minutes, the resuspended cells and DNA/PEI mixture were put together and then transferred into an appropriate container which was placed in a shaking device (37°C, 5% CO₂). After a 3 hours incubation time 800 µL of Ex-Cell Medium, supplemented with 6 mM L-Glutamine, 1.25 mM valproic acid and 12.5% Pepsoy (50 g/L), was added for every mL of final Production volume. After 24 hours, 70 µL of feed solution was added for every mL of final production volume. After 7 days or when the cell viability was equal or lower than 70%, the cells were separated from the supernatant by centrifugation and sterile filtration. The antibodies were purified by an affinity step and one polishing step size exclusion chromatography.

For the affinity step the supernatant was loaded on a protein A column (HiTrap Protein A FF , 5 mL, GE Healthcare) equilibrated with 6 CV 20 mM sodium phosphate, 20 mM sodium citrate, pH 7.5. After a washing step with the same buffer the antibody was eluted from the column by step elution with 20 mM sodium phosphate, 100 mM sodium chloride, 100 mM Glycine, pH 3.0. The fractions with the desired antibody were immediately neutralized by 0.5 M Sodium Phosphate, pH 8.0 (1:10), pooled and concentrated by centrifugation. The concentrate was sterile filtered and processed further by size exclusion chromatography.

For the size exclusion step the concentrated protein was injected in a XK16/60 HiLoad Superdex 200 column (GE Healthcare), and 20 mM Histidine, 140 mM Sodium Chloride, pH 6.0 with or without Tween20 as formulation buffer. The fractions containing the monomers were pooled, concentrated by centrifugation and sterile filtered into a sterile vial.

Determination of the antibody concentration was done by measurement of the absorbance at 280 nm, using the theoretical value of the absorbance of a 0.1% solution of the antibody. This value was based on the amino acid sequence and calculated by GPMAW software (Lighthouse data).

Purity and monomer content of the final protein preparation was determined by CE-SDS (Caliper LabChip GXII system (Caliper Life Sciences)) resp. HPLC (TSKgel G3000 SW XL analytical size exclusion column (Tosoh)) in a 25 mM potassium phosphate, 125 mM Sodium chloride, 200 mM L-arginine monohydrochloride, 0.02 % (w/v) Sodium azide, pH 6.7 buffer.

Figure 3 depicts the CE-SDS graphs (non-reduced (top) and reduced (bottom)) of the final protein preparations after Protein A (PA) affinity chromatography and size exclusion chromatographic (SEC) purification steps (A) pSCHLI333-TCB CLC, (B) pSCHLI372-TCB CLC, (C) pSCHLI373-TCB CLC. PA affinity chromatography and SEC purification steps applied to pSCHLI333-TCB CLC antibody resulted in a purity of 89.2% and 100% of monomer content (A), a purity of 88.5% and 100% of monomer content for pSCHLI372-TCB CLC antibody (B), and a purity of 91.8% and 100% of monomer content for pSCHLI372-TCB CLC antibody (C). Table 10 summarizes the properties of pSCHLI333-TCB CLC, pSCHLI372-TCB CLC, and pSCHLI373-TCB CLC antibodies following PA affinity chromatography and SEC purification steps. In all BCMA-TCB CLC antibodies, >88% purity and 100% monomer content were consistently reached. The overall results clearly demonstrate that advantages in production/purification features could be achieved with using a common light chain (CLC) to TCB antibodies and that only two purification steps (i.e PA affinity chromatography and SEC) were required to achieve already high quality protein preparations with very good developability properties.

**Table 10: Production/purification profile of BCMA-TCB CLC antibodies following protein A affinity chromatography and size exclusion chromatography purification steps**

| | pSCHLI333-TCB CLC | pSCHLI372-TCB CLC | pSCHLI373-TCB CLC |
|---|---|---|---|
| Purity (%) | 89.2 | 88.5 | 91.8 |
| Yield (mg/L) | 4.2 | 4.1 | 2.73 |
| Titer (mg/L) | 26.9 | 21.2 | 37.1 |
| Monomer (%) | 100 | 100 | 100 |

### Example 7: Binding of BCMA-TCB CLC antibodies to BCMA-positive multiple myeloma cell lines (flow cytometry)

BCMA-TCB CLC antibodies (pSCHLI333, pSCHLI372, pSCHLI373) were analyzed by flow cytometry for binding to human BCMA on BCMA^{hi}-expressing H929 cells. MKN45 (human gastric adenocarcinoma cell line that does not express BCMA) was used as negative control. Briefly, cultured cells are harvested, counted and cell viability was evaluated using ViCell. Viable cells are then adjusted to 2 x 10⁶ cells per ml in BSA-containing FACS Stain Buffer (BD Biosciences). 100 µl of this cell suspension were further aliquoted per well into a round-bottom 96-well plate and incubated with 30 µl of the BCMA-TCB CLC antibodies or corresponding TCB control for 30 min at 4°c. All BCMA-TCB CLC antibodies (and TCB controls) were titrated and analyzed in final concentration range between 0.12 - 500 nM. Cells were then centrifuged (5 min, 350 x g), washed with 120µl/well FACS Stain Buffer (BD Biosciences), resuspended and incubated for an additional 30 min at 4°C with fluorochrome-conjugated PE-conjugated AffiniPure F(ab')2 Fragment goat anti-human IgG Fc Fragment Specific (Jackson Immuno Research Lab; 109-116-170). Cells were then washed twice with Stain Buffer (BD Biosciences), fixed using 100 ul BD Fixation buffer per well (#BD Biosciences, 554655) at 4°C for 20 min, resuspended in 120 µl FACS buffer and analyzed using BD FACS CantoII. As depicted in Figure 4, the mean fluorescence intensity of BCMA-TCB CLC antibodies were plotted in function of antibody concentrations; (A) pSCHLI372-TCB CLC and pSCHLI373-TCB CLC on H929 cells; (B) pSCHLI372-TCB CLC and pSCHLI373-TCB CLC on MKN45 cells. BCMA-TCB CLC antibodies (pSCHLI333, pSCHLI372, pSCHLI373) antibodies did not bind to BCMA-negative and CD3-negative MKN45 cells at concentrations below 100 nM. When applicable, EC50 were calculated using Prism GraphPad (LaJolla, CA, USA) and EC50 values denoting the antibody concentration required to reach 50% of the maximal binding for the binding of anti-BCMA/anti-CD3 TCB antibodies to H929 cells are summarized in Table 11.

**Table 11: EC50 values for binding of BCMA-TCB CLC antibodies to H929 cells**

| **Anti-BCMA/anti-CD3 TCB molecules** | **EC50 (nM)** | **EC50 (µg/ml)** |
|---|---|---|
| pSCHLI372-TCB CLC | 344.8 | 65.9 |
| pSCHLI373-TCB CLC | no EC50 value | no EC50 value |

### Example 8: Binding of BCMA-TCB CLC antibodies to CD3-positive Jurkat T cell line (flow cytometry)

BCMA-TCB CLC antibodies (pSCHLI333, pSCHLI372, pSCHLI373) were also analyzed by flow cytometry for their binding properties to human CD3 expressed on human leukemic T cells Jurkat (ATCC TIB-152). Jurkat T cells were cultured in RPMI supplemented with 10% heat-inactivated FCS. Briefly, cultured cells were harvested, counted and cell viability was evaluated using ViCell. Viable cells were then adjusted to 2 x 10⁶ cells per ml in FACS Stain Buffer (BD Biosciences) containing 0.1% BSA. 100 µl of this cell suspension were further aliquoted per well into a round-bottom 96-well plate. 30 µl of BCMA-TCB CLC antibodies or corresponding TCB control were added to the cell-containing wells to obtain final concentrations of 0.12 nM to 500 nM. BCMA-TCB CLC antibodies and control IgG were used at the same molarity. After incubation for 30 min at 4°C, cells were centrifuged (5 min, 350 x g), washed twice with 150 µl/well BSA-containing FACS Stain Buffer (BD Biosciences), then cells were fixed using 100 ul BD Fixation buffer per well (#BD Biosciences, 554655) at 4°C for 20 min, resuspended in 120 µl FACS buffer and analyzed using BD FACS CantoII. Binding of BCMA-TCB CLC antibodies to T cells were evaluated and the median fluorescence intensity was determined gated on CD3-expressing Jurkat T cells and plotted in histograms or dot plots. Figure 5 shows the median fluorescence intensity for BCMA-TCB CLC antibodies (pSCHLI333, pSCHLI372, pSCHLI373) binding to Jurkat T cells (A) or MKN45 cells (B) and plotted in function of antibody concentration. EC50 values and maximal binding of anti-BCMA/anti-CD3 TCB antibodies to CD3-positive Jurkat T cells were not reached. Isotype control antibody did not bind to Jurkat T cells and BCMA-TCB CLC antibodies (pSCHLI333, pSCHLI372, pSCHLI373) antibodies did not bind to BCMA-negative and CD3-negative MKN45 cells at concentrations below 100 nM.

### Example 9: Activation of human T cells upon binding of BCMA-TCB CLC antibodies to CD3-positive T cells and BCMA-positive multiple myeloma cell lines (Flow cytometry)

BCMA-TCB CLC antibodies (pSCHLI372, pSCHLI373) were analyzed by flow cytometry for their ability to induce T cell activation by evaluating the surface expression of the early activation marker CD69, or the late activation marker CD25 on CD4⁺ and CD8⁺ T cells in the presence or absence of human BCMA-expressing MM cells. Briefly, BCMA-positive H929 cells were harvested with Cell Dissociation buffer, counted and checked for viability. Cells were adjusted to 0.3 x 10⁶ (viable) cells per ml in modified RPMI-1640 medium, 100 µl of this cell suspension were pipetted per well into a round-bottom 96-well plate (as indicated). 50 µl of the (diluted) BCMA-TCB CLC antibodies were added to the cell-containing wells to obtain a final concentration of 0.012 pM - 100 nM. Human PBMC effector cells were isolated from fresh blood of a healthy donor and adjusted to 6 x 10⁶ (viable) cells per ml in modified RPMI-1640 medium. 50 µl of this cell suspension was added per well of the assay plate to obtain a final E:T ratio of PBMC to myeloma tumor cells of 10: 1. To analyze whether the BCMA-TCB CLC antibodies were able to activate T cells specifically in the presence of target cells expressing human BCMA, wells were included that contained 3 to 10 nM of the respective BCMA-TCB CLC antibodies molecules, as well as PBMCs, but no target cells. After 48 h incubation at 37°C, 5% CO₂, cells were centrifuged (5 min, 350 x g) and washed twice with 150 µl/well PBS containing 0.1% BSA. Surface staining for CD4 (mouse IgGl,K; clone RPA-T4), CD8 (mouse IgGl,K; clone HIT8a; BD #555635), CD69 (mouse IgGl; clone L78; BD #340560) and CD25 (mouse IgGl,K; clone M-A251; BD #555434) was performed at 4°C for 30 min, according to the supplier's suggestions. Cells were washed twice with 150 µl/well PBS containing 0.1% BSA and fixed for 15 min at 4°C, using 100 µl/well fixation buffer (BD #554655). After centrifugation, the samples were resuspended in 200 µl/well PBS with 0.1% BSA and analyzed using a FACS CantoII machine (Software FACS Diva). Figure 6 depicts the expression level of the early activation marker CD69 and the late activation marker CD25 on CD4⁺ and CD8⁺ T cells after 48 hours of incubation (representative results from two independent experiments). pSCHLI372-TCB CLC and pSCHLI373-TCB CLC antibodies induced an up-regulation of CD69 and CD25 activation markers in a concentration-dependent and specific manner in the presence of BCMA-positive target cells. No activation of CD4⁺ and CD8+ T cells was observed when human PBMCs were treated with DP47-TCB control antibody, suggesting that despite binding to CD3 on the T cells T-cell activation does not occur when the TCB antibody does not bind to BCMA-positive target cells.

### Example 10: Redirected T-cell cytotoxicity of BCMA^{hi}-expressing H929 myeloma cells induced by anti-BCMA/anti-CD3 T cell bispecific antibodies (colorimetric LDH release assay)

BCMA-TCB CLC antibodies (pSCHLI372, pSCHLI373) were also analyzed for their potential to induce T cell-mediated apoptosis in BCMA-high expressing MM cells upon crosslinking of the construct via binding of the antigen binding moieties to BCMA on cells. Briefly, human BCMA-expressing H929 multiple myeloma target cells were harvested with Cell Dissociation Buffer, washed and resuspended in RPMI supplemented with 10% fetal bovine serum (Invitrogen). Approximately, 30,000 cells per well were plated in a round-bottom 96-well plate and the respective dilution of the construct was added for a desired final concentration (in triplicates); final concentrations ranging from 0.12 pM to 100 nM. For an appropriate comparison, all TCB constructs and controls were adjusted to the same molarity. Human total T cells (effector) were added into the wells to obtain a final E:T ratio of 5:1. When human PBMC were used as effector cells, a final E:T ratio of 10:1 was used. Negative control groups were represented by effector or target cells only. As a positive control for the activation of human pan T cells, 1 µg/ml PHA-M (Sigma #L8902) was used. For normalization, maximal lysis of the H929 MM target cells (= 100%) was determined by incubation of the target cells with a final concentration of 1% Triton X-100, inducing cell death. Minimal lysis (= 0%) was represented by target cells co-incubated with effector cells only, i.e. without any T cell bispecific antibody. After 20-24h or 48h incubation at 37°C, 5% CO₂, LDH release from the apoptotic/necrotic MM target cells into the supernatant was then measured with the LDH detection kit (Roche Applied Science), following the manufacturer's instructions. The percentage of LDH release was plotted against the concentrations of BCMA-TCB CLC antibodies in concentration-response curves. The EC50 values were measured using Prism software (GraphPad) and determined as the TCB antibody concentration that results in 50% of maximum LDH release. As shown in Figure 7, BCMA-TCB CLC antibodies, pSCHLI372-TCB CLC (A, B) and pSCHLI373-TCB CLC (B) induced a concentration-dependent killing of BCMA-positive H929 myeloma cells as measured by LDH release. The killing of H929 cells was specific since DP47-TCB control antibody which does not bind to BCMA-positive target cells did not induce LDH release, even at the highest concentration tested of 100 nM. Table 12 summarizes the EC50 values for redirected T-cell killing of BCMA-positive H929 cells induced by BCMA-TCB CLC antibodies.

**Table 12: EC50 values for redirected T-cell killing of H929 cells induced by BCMA-TCB CLC antibodies**

| **Anti**-**BCMA**/**anti**-**CD3 TCB molecules** | **EC50 (pM)** | **EC50 (µg/ml)** |
|---|---|---|
| pSCHLI333-TCB CLC | 980 | 0.19 |
| pSCHLI372-TCB CLC (Experiment 1) | 450 | 0.08 |
| pSCHLI373-TCB CLC | 1590 | 0.31 |
| pSCHLI372-TCB CLC (Experiment 2) | 900 | 0.17 |

### Example 11: Redirected T-cell cytotoxicity of BCMA^{med/lo}-expressing U266 myeloma cells induced by BCMA-TCB CLC antibodies (LDH release assay)

BCMA-TCB CLC antibodies (pSCHLI372, pSCHLI373) were analyzed for their ability to induce T cell-mediated apoptosis in BCMA^{med/lo}-expressing MM cells upon crosslinking of the construct via binding of the antigen binding moieties to BCMA on cells. Briefly, human BCMA^{med/lo}-expressing U266 multiple myeloma target cells were harvested with Cell Dissociation Buffer, washed and resuspended in RPMI supplemented with 10% fetal bovine serum (Invitrogen). Approximately, 30,000 cells per well are plated in a round-bottom 96-well plate and the respective dilution of the construct was added for a desired final concentration (in triplicates); final concentrations ranging from 0.12 pM to 100 nM. For an appropriate comparison, all TCB constructs and controls were adjusted to the same molarity. Human total T cells (effector) were added into the wells to obtain a final E:T ratio of 5:1. When human PBMC were used as effector cells, a final E:T ratio of 10:1 was used. Negative control groups were represented by effector or target cells only. As a positive control for the activation of human T cells, 1 µg/ml PHA-M (Sigma #L8902) was used. For normalization, maximal lysis of the MM target cells (= 100%) was determined by incubation of the target cells with a final concentration of 1% Triton X-100, inducing cell death. Minimal lysis (= 0%) was represented by target cells co-incubated with effector cells only, i.e. without any T cell bispecific antibody. After 20-24 h incubation at 37°C, 5% CO₂, LDH release from the apoptotic/necrotic MM target cells into the supernatant was then measured with the LDH detection kit (Roche Applied Science), following the manufacturer's instructions. The percentage of LDH release was plotted against the concentrations of BCMA-TCB CLC antibodies in concentration-response curves. The EC50 values were measured using Prism software (GraphPad) and determined as the TCB antibody concentration that results in 50% of maximum LDH release. Table 13 summarizes the EC50 values for redirected T-cell killing of BCMA-positive U266 cells induced by BCMA-TCB CLC antibodies.

**Table 13: EC50 values for redirected T-cell killing of U266 cells induced by BCMA-TCB CLC antibodies**

| **Anti**-**BCMA**/**anti**-**CD3 TCB molecules** | **EC50 (pM)** | **EC50 (µg/ml)** |
|---|---|---|
| pSCHLI372-TCB CLC | 5700 | 1.1 |

## Claims

1. An antibody light chain, comprising as CDRs CDR1L of SEQ ID NO:6, CDR2L of SEQ ID NO:7, and CDR3L of SEQ ID NO:8, for use as common light chain in a bispecific antibody specifically binding to the extracellular domain of human BCMA (further named also as "BCMA") and human CD3 (further named also as "CD3"), wherein the heavy chain of the CD3 binding part comprises as CDRs CDR1H of SEQ ID NO:3, CDR2H of SEQ ID NO:4, and CDR3H of SEQ ID NO:5.

2. The antibody light chain according to claim 1, comprising as variable light chain domain VL a VL of SEQ ID NO:2.

3. The antibody light chain according to claim 1 or 2, comprising as variable light chain of SEQ ID NO:29 for use as common light chain in a bispecific antibody specifically binding to the extracellular domain of human BCMA and human CD3, wherein the heavy chain of the CD3 binding part comprises as CDRs CDR1H of SEQ ID NO:3, CDR2H of SEQ ID NO:4, and CDR3H of SEQ ID NO:5.

4. A bispecific antibody specifically binding to the extracellular domain of human and human CD3, **characterized in** comprising as CDRs of the BCMA binding part a CDR set selected from the group consisting of:
a) CDR1H of SEQ ID NO:18, CDR2H of SEQ ID NO:21, and CDR3H of SEQ ID NO:24
b) CDR1H of SEQ ID NO:19, CDR2H of SEQ ID NO:22, and CDR3H of SEQ ID NO:25
c) CDR1H of SEQ ID NO:20, CDR2H of SEQ ID NO:23, and CDR3H of SEQ ID NO:26
d) CDR1H of SEQ ID NO:35, CDR2H of SEQ ID NO:36, and CDR3H of SEQ ID NO:37

5. The antibody according to claim 4, **characterized in** consisting of two BCMA-Fabs, one CD3 Fab and a Fc part, wherein the first BCMA-Fab and the CD3-Fab are linked via their C-termini to the hinge region of said Fc part and said second BCMA-Fab is linked with its C-terminus to the N-terminus of said first BCMA-Fab. The CD3-Fab and BCMA-Fabs comprise said common light chains for use according to the invention.

6. The antibody according to claim 4, **characterized in** consisting of two BCMA-Fabs and a Fc part, wherein said BCMA-Fabs are linked via their C-termini to the hinge region of said Fc part and said CD3-Fab is linked with its C-terminus to the N-terminus of one BCMA-Fab. Said CD3-Fab and BCMA-Fabs comprise said common light chains for use according to the invention.

7. The antibody according to claim 4, **characterized in** consisting of one CD3-Fab and one BCMA Fab, which is linked via its C-terminus to the hinge region of said Fc part and a BCMA-Fab, which is linked with its C-terminus to the N-terminus of said CD3-Fab. Said CD3-Fab and BCMA-Fab comprise said common light chains for use according to the invention.

8. The antibody according to claim 4, **characterized in** consisting of one BCMA-Fab and one CD3 Fab, wherein said BCMA Fab is linked via its C-terminus to the hinge region of said Fc part and said CD3-Fabis linked with its C-terminus to the N-terminus of said BCMA-Fab. Said CD3-Fab and BCMA-Fab comprise said common light chains for use according to the invention.

9. The antibody according to claim 4, **characterized in** consisting of two BCMA-Fabs, wherein the first BCMA-Fab is linked via its C-terminus to the N-terminus of the second BCMA-Fab, and wherein the second BCMA-Fab is linked via its C-terminus to the N-terminus of the CD3-Fab. The CD3-Fab and both BCMA-Fabs comprise said common light chains for use according to the invention.

10. The antibody according to claim 4, **characterized in** consisting of one BCMA-Fab and one CD3 Fab, wherein the BCMA Fab which is linked with its C-terminus to the N-terminus of the CD3-Fab. The CD3-Fab and BCMA-Fab comprise said common light chains for use according to the invention.

11. A method for the preparation of an a bispecific antibody according to any one of claims 4 to 10 comprising the steps of
a) transforming a host cell with vectors comprising nucleic acid molecules encoding the light chain and heavy chain of an antibody according to any one of claims 4 to 10,
b) culturing the host cell under conditions that allow synthesis of said antibody molecule; and
c) recovering said antibody molecule from said culture.

12. A pharmaceutical composition comprising the antibody according to any one of claims 4 to 10 and a pharmaceutically acceptable excipient.

13. The antibody according to any one of claims 4 to 10 or the pharmaceutical composition of claim 12 for use as a medicament in the treatment of plasma cell disorders like Multiple Myeloma.

14. The antibody light chain for use as common light chain in a bispecific antibody according to claim 1, wherein said bispecific antibody is an antibody according to claims 4 to 10.

15. Use of an antibody light chain, comprising as CDRs CDR1L of SEQ ID NO:6, CDR2L of SEQ ID NO:7, and CDR3L of SEQ ID NO:8, as common light chain in a bispecific antibody specifically binding to the extracellular domain of human BCMA (further named also as "BCMA") and human CD3 (further named also as "CD3"), wherein the heavy chain of the CD3 binding part comprises as CDRs CDR1H of SEQ ID NO:3, CDR2H of SEQ ID NO:4, and CDR3H of SEQ ID NO:5.
